# EUROPEAN PATENT SPECIFICATION

(11) **EP 4 607 190 B1**
(45) Date of publication and mention of the grant of the patent: **14.10.2026**
(21) Application number: 25189579.3
(22) Date of filing: 11.03.2020
(51) Int. Cl.: G01N 33/487

(54) **NANOPORE SENSING DEVICE AND METHOD**
NANOPORENMESSVORRICHTUNG UND -VERFAHREN
DISPOSITIF ET PROCÉDÉ DE DÉTECTION DE NANOPORES

(30) Priority: 12.03.2019 US 201962817211 P
(43) Date of publication of application: 27.08.2025
(62) Document number(s) of the earlier application(s) in accordance with Art. 76 EPC: 20715941.9 / 3 938 779
(73) Proprietor: Oxford Nanopore Technologies PLC, Oxford OX4 4DQ (GB)
(72) Inventor: XIE, Ping, Cambridge, 02139 (US); MILLIS, Justin, Cambridge, 02139 (US); HEALY, Ken, Cambridge, 02139 (US)
(74) Representative: J A Kemp LLP

(56) References cited:
- CN-A- 103 995 035
- US-A1- 2013 270 521
- US-A1- 2014 243 214
- US-A1- 2014 318 964

## Description

The invention relates, in general, to a device for nanopore sensing, having an array of nanopore structures configurable as nanopore sensors, as well as methods for operating nanopore sensors or fabricating an array of nanopore structures.

Nanopore sensors have been developed for sensing a wide range of species, including single molecules such as polymer molecules. A known nanopore sensor device is a MinION^{™}, manufactured and sold by Oxford Nanopore Technologies Ltd. The nanopore-based sensing therein employs the measurement of ionic current flow through a biological nanopore located in a highly resistive amphiphilic membrane. The MinION^{™} has an array of nanopore sensors. As a molecule, such as a polymer analyte e.g. DNA, is caused to translocate a nanopore, measurement of the fluctuations in ionic current may be used to determine the sequence of the DNA strand. Nanopore devices for detection of analytes other than polynucleotides such as proteins are also known from WO2013/123379.

An alternative to biological nanopore devices, such as MinION^{™}, are solid state nanopore devices. Fig. 1 shows a portion of a single sensor device 2 with a solid-state nanopore 4 disclosed in WO2016/127007, hereby incorporated by reference in its entirety, in which an analyte 6 passes through a body 8 from a cis reservoir 10, through the solid-state nanopore 4 and into a fluidic passage 12; a signal is read via a sensor 16 located close to the solid-state nanopore 4. Electrodes 18 are provided in the cis and trans reservoirs 10, 14 for inducing the analyte 6 through the solid-state nanopore 4.

The performance of solid-state nanopore sensors is limited by the sensing components, manufacturing techniques and their tolerances, which can occur as a result of variation in the formation of the nanopore or the assembly of the sensor. These and other factors detriment the bandwidth, sensitivity and ability to control such nanopore sensors.

CN 103 995 035 A discloses a multi-grid graphene field-effect tube structure for detection of a base sequence. When a base to be detected passes through a graphene nanopore, voltage modulation is carried out on a metal gate electrode and base signals are detected at the ends of metal source electrodes so as to realize the recognition of the base sequence.

The present invention is concerned with overcoming problems associated with implementing a nanopore sensor array having a plurality of nanopore sensors.

The present inventors sought to improve upon known nanopore sensing devices by providing the ability to control the movement of an analyte while also improving the measurement accuracy by mitigating factors that impede on the measurement, such as noise caused by parasitics and contaminated sensing components. Moreover, the improved devices allow the nanopore structures, and nanopore sensors implemented therefrom, to be formed in large arrays in an efficient manner without inhibiting the control or performance of the array.

The invention provides a device having an array of nanopore structures according to claim 1.

The sheet can be a substantially planar array of nanopore structures. When the nanopore structures are provided with a nanopore they can function as nanopore sensors. The sheet can be configured in the device to separate an analyte reservoir and an outlet chamber. The analyte reservoir and the outlet chamber can accommodate a fluid. The passages can be filled with a fluid and provide a fluid connection between the analyte reservoir and theoutlet chamber.

Configuring the nanopore layer and the base layer as separate layers can improve the scalability of the sheets. The layers can facilitate assembly of the device, thus reducing the cost of manufacture. The layering of the sheet can bring together the components of the nanopore structure in an efficient manner. Moreover, by having the different components of the nanopore structure on different layers can enable the formation or configuration of those components to be optimised. It is often the case that the process used in the fabrication of one component is incompatible or detrimental to the fabrication of another component. Furthermore, the optimal material for forming one component can be different from the optimal material for forming other components. By way of example, the array of nanopores and/or an array of wells of the nanopore can be formed separately from the base layer. The nanopore layer and base layer can comprise different materials. The separate layers can enable the components of the nanopore structure to be optimally configured and/or located.

The provisions of layers can enable a layer to be replaceable. The nanopore layer can be removably attachable. In this way a nanopore layer can be replaced with replacement nanopore such that the device can be recycled should, for example, the nanopore layer become contaminated.

Each nanopore structure of the sheet is defined by the passage. The various components of the nanopore structure i.e. the nanopore or nanopore well, the electrical transduction element and the channel form the passage.

A nanopore is configured over the well of the nanopore layer, and in so doing this nanopore over the well also forms an element of the passage.

The electrical transduction element in each passage is disposed between the nanopore layer and at least a portion of the channel. The electrical transduction element can be configured with a connection for measuring electrical potential of the fluid at the location of the electrical transduction element when the structure is provided with a nanopore and a fluid is provided in the passage.

The electrical transduction element can develop a characteristic that is indicative of the fluidic electrical potential at the electrical transduction element in that passage, via fluid in the passage that connects the cis and trans. The electrical transduction element can be an electrical connection. It can be located in the cis or the trans reservoir, on a surface of the nanopore structure, at a position within the passage, or other location within the nanopore structure.

The electrical transduction element can be a device or region of a device and/or circuit, a wire, or combination of circuit elements, that senses the fluidic electrical potential at the electrical transduction element of the device. Additionally, or alternatively, the circuit can be provided as a transduction element to develop a signal indicative of local electrical potential.

As described, the device can have an analyte reservoir and an outlet chamber separated, at least in part, by the sheet. The analyte reservoir can function as a cis and hold an analyte to be analysed by the nanopore structure when provided with a sensor. The passages of the nanopore structures of the array connect the analyte reservoir to the outlet chamber. The interface between the analyte reservoir and the outlet chamber can be the passage or, more specifically, the nanopore in a nanopore sensor i.e. a nanopore structure provided with a nanopore.

The device can have drive electrodes connected in the analyte reservoir and outlet chamber to impose an electrical potential difference across the array of passages between the analyte reservoir and outlet chamber.

The sheet can be substantially planar. The surfaces of the sheet, which is the structure incorporating the array of nanopore structures, can have a cis-surface on the nanopore layer for facing an analyte reservoir and defining a cis-plane, and a trans-surface of the base layer for facing an outlet chamber and defining a trans-plane. The array of electrical transduction elements is embedded within the sheet between the cis-plane and the trans-plane. The electrical transduction elements of the array are sandwiched between the nanopore layer and the base layer.

Each nanopore structure of the array has a well formed at a first end of the passage. A nanopore is configured at the first end of each well. The electrical transduction element is configured on the opposite side of the well to the nanopore. The well can be larger in size that the nanopore and increase the volume of fluid surrounding the nanopore. To be clear, the diameter of the well can be greater than the diameter of the nanopore. The nanopore can reside in a membrane that spans the well. The membrane can be a solid-state membrane, an amphiphilic membrane or a lipid bilayer. The nanopore defines a portion of the passage. Ingress and egress from the well are via the nanopore and a well outlet.

The well can be configured for supporting a fluid membrane such as a polymer membrane or lipid bilayer. The nanopore layer can be fabricated from a different material from the base layer. By using a different material for the nanopore layer a material can be selected to have a surface energy that optimises the formation of a membrane across the well for supporting a nanopore.

The electrical transduction element can be a sensor electrode. The sensor electrode can be directly connectable to the base or gate of a transistor device for measuring variations in electrical potential of the fluid at the location of the electrical transduction element when a fluid is provided in the passage. As described herein, a nanopore structure provided with a nanopore forming a portion of the passage functions as a nanopore sensor, and the sensing is performed by the electrical transduction element.

The electrical transduction elements of the nanopore structures of the array can be connected to an edge-connector or wire-bond. The connector can provide a connection to a measurement circuit off-sheet i.e. separate from the array of nanopore structures. The connector can be connected to a via that leads to a connection at the edge of the sheet, for subsequent connection to a measurement circuit off-sheet. The transistor device can be a field effect transistor.

The sheet has been described thus far having a nanopore layer and base layer. The electrical transduction element can be a layer within the sheet or can have elements sandwiched between layers. The sheet of the device can, however, further comprise a sense layer having an array of the electrical transduction elements, wherein said sense layer is sandwiched between the nanopore layer and the base layer. The electrical transduction element can be formed upon the sense layer. The electrical transduction element can have an exposed portion for connection to a fluid in the passage and an embedded portion embedded within the sheet. Additionally, or alternatively, the electrical transduction element can have a connection portion for connection to a measurement circuit separate from the sheet. By incorporating the electrical transduction element in or upon the sense layer this enables the formation of electrical transduction element to be separate from the manufacture of the other layers. The sense layer can be fabricated using a different material, process and/or techniques from the other layers.

The electrical transduction element can cover, at least in part, a wall of the passage. The electrical transduction element can cover, in cross-section, a portion of a wall of the channel. The electrical transduction element can form an annulus around the passage and/or the base of a well or cavity within the passage.

The electrical transduction elements can be formed on one surface of the sense layer. The sense layer can be sandwiched between the base layer and the nanopore layer with the electrical transduction elements aligned with the nanopore or wells of the nanopore layer and the channels of the base layer. When aligned, the face of the sense layer can have the electrical transduction elements exposed to the nanopore layer, such that the nanopore layer is formed or placed upon the surface having the electrical transduction elements; in this arrangement the electrical transduction element can be said to face the nanopore layer. Alternatively, when aligned, the face of the sense layer can have the electrical transduction elements exposed to the base layer, such that the electrical transduction element is formed or placed upon the surface of the base layer; in this arrangement the electrical transduction element can be said to face the base layer.

The electrical transduction element can form, at least in part, the surface of the sense layer around the passage and have an exposed portion arranged to face the outlet chamber. The exposed portion can form part of a wall of a cavity formed in the sense layer between the well and the channel. The cavity enables a greater area of the sensor electrode to be exposed to fluid in the passage. This can improve the sensitivity of the sensor electrode.

The electrical transduction element can have an aperture forming a portion of the passage and exposed portion, wherein in cross-section, the ratio of the size of the exposed portion of the electrical transduction element to the size of the aperture is 1:1. The ratio can be about 5:1.

The electrical transduction element can have an aperture forming a portion of the passage and exposed portion, wherein in plan-view, the ratio of the size of the exposed portion of the electrical transduction element to the size of the aperture is 1:1. The electrical transduction element can have an aperture forming a portion of the passage and exposed portion, wherein the ratio is about 5:1. The aperture can be circular.

The electrical transduction element can have a large exposed area to increase the exposure to a fluid in the passage to increase the sensitivity of the element to fluctuations in voltage caused by an analyte passing over, or through, a nanopore in the passage.

The sense layer can incorporate an electronic circuit for each nanopore structures. The circuit can be connected to the electrical transduction element for modifying and/or processing the signals received therefrom. By incorporating an electronic circuit within each nanopore structure then signals from the electrical transduction elements can be processed locally to inhibit any attenuation of information in the signal derived therefrom and/or inhibit any detriment to that signal from noise. Each electrical circuit in the respective nanopore structure can process signals from the sensor electrode prior to said processed signal being communicated off-sheet for further processing and/or analysis. By incorporating the circuit in the sense layer, the circuit can be embedded in the nanopore structure. The circuit can occupy the same footprint as the nanopore structure such that the nanopore structure can be considered as an active pixel. A nanopore structure having its own circuit can complement the improved control mechanism disclosed herein by having a control signal generated and applied locally, thus minimising the influence of the control signal upon other nanopore structures of the array. The circuit within the sense layer of the nanopore circuit can be a compensation circuit.

The electronic circuit can be configured to detect resistance changes at a nanopore in a respective passage when an analyte passes through the nanopore, or adjacent said nanopore. The circuit can detect a resistance change detected through the fluid in the sensor.

While the device has been described as suitable for sensing an analyte it should be appreciated that the analyte is one that can be measured using a nanopore. By way of example the analyte can be a protein, polymer, polynucleotide or the like.

The electronic circuit can detect resistance changes at the nanopore when a polymer passes through the nanopore and converts it to a voltage signal and amplifies said voltage signal. The electronic circuit can filter the signal. The electronic circuit can sample and/or digitise signals obtained from an electrical transduction element.

Each nanopore structure can have a plurality of electrical transduction elements corresponding to each respective nanopore structure. Similarly, each nanopore structure can have a plurality of circuits corresponding to each respective nanopore structure and/or electrical transduction element provided in that nanopore structure. Each of the electrical transduction elements and/or circuits can be configured in an addressable array. Each nanopore structure can have two or more sensor electrodes. Two or more sensor electrodes can be connected to a single circuit within the nanopore structure or each sensor electrode could be connected to its own circuit.

The array of nanopore structures can be connected to an architecture for enabling readout from each nanopore structure individually (which may be referred to as pixels) in a matrix array. Each nanopore structure can have a row number and column number.

Each electrical transduction element can have a dedicated electronic circuit, and each electrical transduction element and electronic circuit can be located in a footprint. The footprint can be a pixel such that the nanopore structures are tessellated in the array.

While each nanopore structure of the array has an electrical transduction element and, optionally a circuit and/or a control terminal, in light of the teaching herein it can be appreciated that each nanopore structure can have a plurality of electrical transduction elements and/or a plurality of circuits, each circuit providing one or more functions. By way of example, a nanopore structure can have an electrical transduction element for sensing, and a corresponding circuit to process signals from that electrical transduction element and have a second electrical transduction element adapted for applying a control signal to the passage in the nanopore structure, said second electrical transduction element having a circuit for controllably applying said control signal.

It follows that a plurality of electrical transduction elements is arranged in a module having a plurality of respective nanopore structures. The module can have a common dedicated electronic circuit, and each of the electrical transduction elements and electronic circuit are located in a footprint occupied by the plurality of nanopore structures. The module can have, for example, four nanopore structures, each having a respective electrical transduction element, wherein each electrical transduction element is connected to a common circuit. The common circuit can be addressably connected to an external off-structure or off-sheet electronic circuit.

The plurality of nanopore structures can be arranged in a two-dimensional matrix. The plurality of nanopore structures can be arranged in a tessellated pattern.

The electrical transduction element can be connected to the base or gate of a transistor for sensing. The transistor can be a field effect transistor.

Each of the nanopore structures can have a control terminal for applying a control signal to alter the electrical potential difference across the respective nanopore structure. The control terminal can be switchably connectable to the electrical transduction element. The control terminal can be switchably connectable to a power supply to change the configurable voltage level imposed upon the pore. The electrical transduction element and connection for measuring electrical potential of the fluid can be switchably isolatable from the control signal. The electrical transduction element and control electrode can be physically separate. At least a portion of the electrical transduction element and at least a portion of the control electrode can extend in the same plane. At least a portion of the electrical transduction element and at least a portion of the control electrode form, at least in part, the base of a well. At least a portion of the electrical transduction element and at least a portion of control electrode can extend perpendicularly from one another. At least a portion of the control electrode can be configured, at least in part, in the channel. The surface area of the electrical transduction element exposed to the passage can be less than the surface area of the control electrode exposed to the passage.

The device herein can be configured with a conductive guard configured in at least one of the nanopore layer, base layer or sensing layer. The conductive guard can extend between at least one of the electrical transduction element, and signal conductors connected thereto, and parasitic conductive elements in the nanopore layer, base layer or sense layer to inhibit parasitic capacitance from influencing the measurements obtained from the connection. A buffered version of the input signal can be applied to the guard conductor. As a result, there is no voltage difference across the capacitance from the input signal conductor to the conductive substrate.

The conductive guard can include, at least in part, an insulated guard conductor having and an insulating layer. The conductive guard can be configured to extend, at least in part, between the base layer and the channel.

In fabricating a device having nanopore structures for sensing an analyte, the method of fabrication comprises the steps of claim 14.

Aligning the nanopore layer, base layer and array of electrical transduction elements includes sandwiching the array of electrical transduction elements between the nanopore layer and the base layer. The step of sandwiching can include bonding or otherwise connecting the two layers.

The method can further comprise forming cavities adjacent at least a portion of each of the electrical transduction elements. These cavities can increase the area of the element exposed to a fluid in the passage.

The method can further comprise: forming the array of electrical transduction elements on a sense layer; and sandwiching the sense layer between the nanopore layer and the base layer.

The method can further comprise: forming the array of electrical transduction elements on a sense layer; fabricating an array of electronic circuits in the sense layer, said circuits connected to respective electrical transduction elements for modifying and/or processing the signals received therefrom; and sandwiching the sense layer between the nanopore layer and the base layer.

The method can further comprise arranging the electrical transduction element to have: (i) an exposed portion for connection to a fluid in the passage, and (ii) an embedded portion embedded within the structure, and/or (iii) a connection portion for connection to a measurement circuit separate from the structure.

The method can further comprise forming a conductive guard in at least one of the nanopore layer, base layer or sense layer, said conductive guard configured to extend between at least one of the electrical transduction elements and signal conductors connected thereto and parasitic conductive elements in at least one of the nanopore layer, base layer or sense layer to inhibit parasitic capacitance from influencing the measurements obtained from the connection.

The method can further comprise providing for each nanopore structure a buffer, said buffer connecting the output of the electrical transduction element of that nanopore structure to a conductive guard.

The method can further comprise providing amphiphilic membranes in each of the nanopore structures of the array and inserting a biological nanopore in said membranes.

The method can include removably attaching the structure and/or removing the nanopore layer and replacing it with another nanopore layer. **In** this manner the device can be recycled.

Embodiments of the invention are discussed below, by way of non-limitative example only, with reference to the drawings in which:

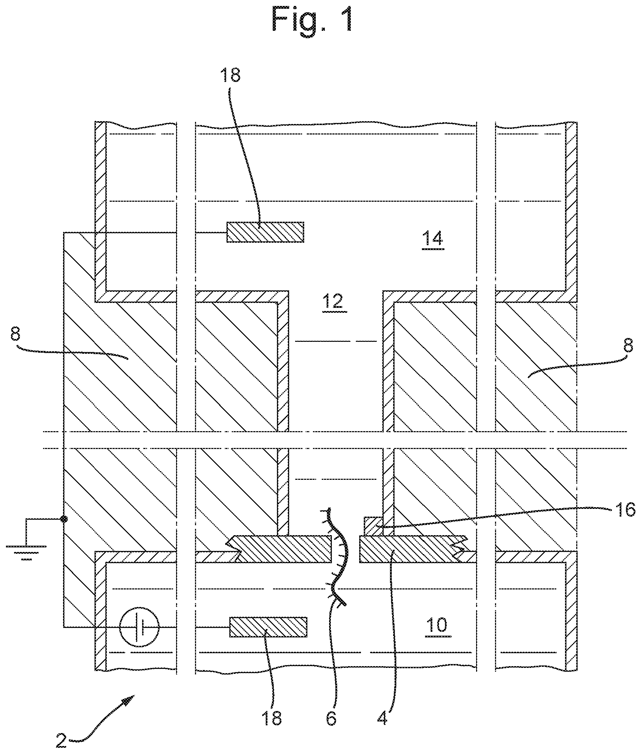

Fig. 1 is a cross sectional view of a known nanopore sensor;
Fig. 2 is a cross-section of a single sensor electrode and corresponding biological nanopore within a nanopore array configured in a structure, and connected via a wire to a measurement circuit;
Fig. 3(a) is an alternative cross-section of a single sensor electrode, and corresponding biological nanopore, the sensor electrode configured on a sensor layer that is sandwiched between a nanopore layer and a base layer within a portion of a structure of a nanopore structure array, wherein the sensor electrode is connected via a wire to an electronic circuit, while Fig. 3(b) is comparable to Fig. 3(a) wherein the sense layer incorporates electronic circuitry;
Fig. 4(a) is a schematic view of the layout of a nanopore sensor of Fig. 3(b) indicating the position of the well with respect to the electronic circuit;
Fig. 4(b) shows two adjacent sensor electrodes before a nanopore is added;
Figs. 4(c) and 4(d) are examples of portions of a structure having an array of the nanopore sensors of Fig. 4(a);
Fig. 4(e) is a schematic showing how a structure can be arranged to separate two chambers in a device;
Fig. 4(f) is an alternative layout of four nanopore structures;
Figs. 5(a) to 5(d) show various electronic circuits implementing electronic functions that interface with an electrical transduction element;
Figs. 6(a) to 6(e) are cross-sectional schematics of the sensor electrode and control electrode configured in a nanopore sensor of the array;
Fig. 7(a) shows, respectively, two schematic circuits illustrating the parasitic capacitance in the array with and without guarding, while Fig. 7(b) is an alternative cross-sectional view to that shown in Fig. 2, in which a guard conductor is configured in the structure and connected via an additional wire to the measurement circuit, and Figs. 7(c) and 7(d) show alternative cross-sections in which guarding is configured; and
Figs. 8 and 9 are side views of respective examples of a device including an array of nanopore structures.

The embodiments include corresponding components, typically labelled by a common reference numeral. For purposes of clarity, the description of corresponding components is not repeated but applies generally to all embodiments, except where the context demands otherwise. Not every component is labeled in every figure, nor is every component of each embodiment of the invention shown where illustration is not necessary to understand the invention.

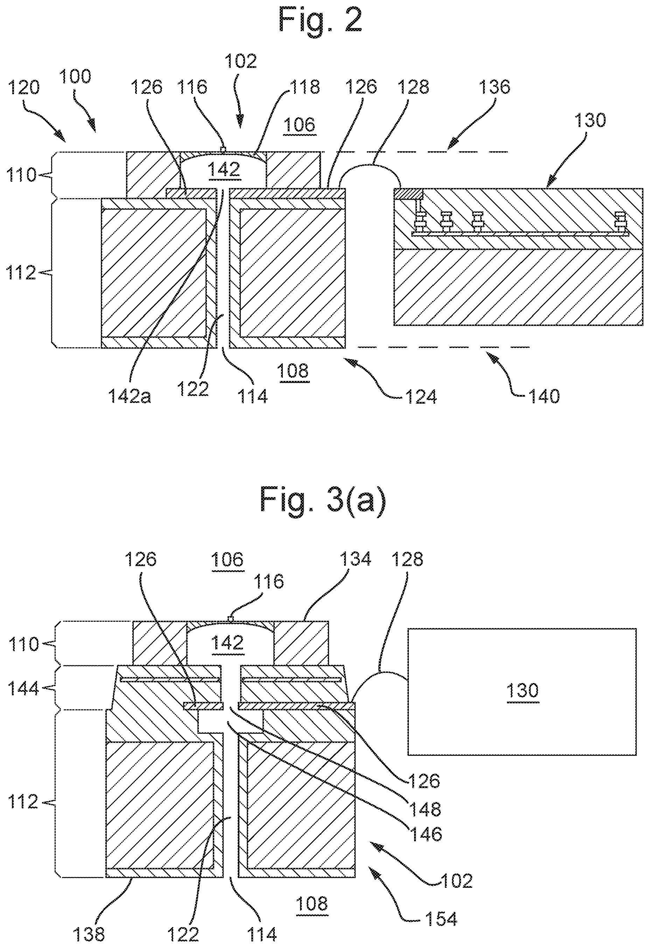

Figs. 2 to 4(a) are sectional views of a portion of a structure 100 having a nanopore structure incorporated therein. The structure 100 has an array of nanopore structures, each nanopore structure adapted to support a nanopore 116. The nanopore structures of the device can function as nanopore sensors when configured with a nanopore. A nanopore sensor 102 herein is nanopore structure having a nanopore.

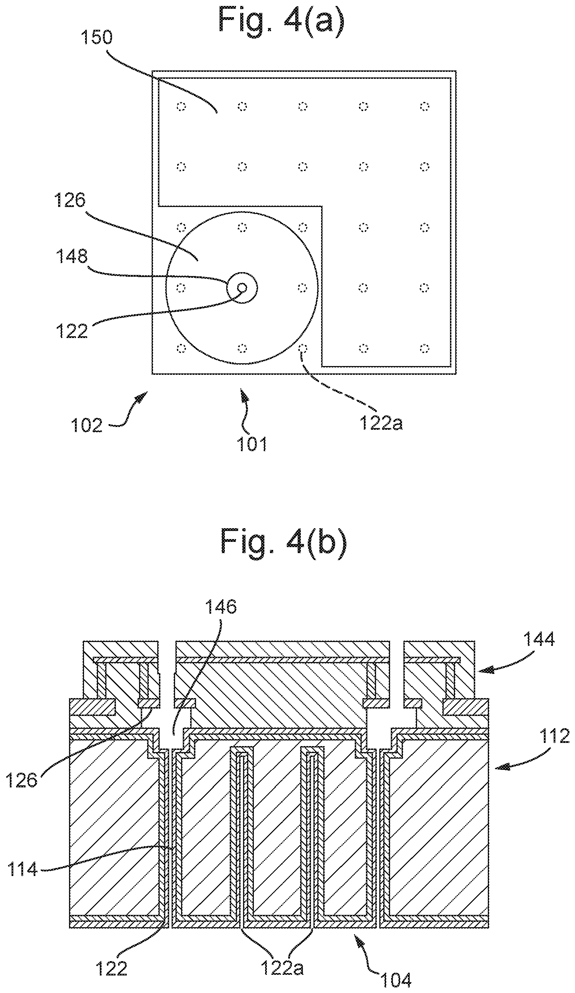

Figs. 4(b) to 4(f) illustrate that a plurality of the nanopore sensors 102 shown in Figs. 2 to 4(a) is arranged as part of an array of nanopore structures 104. Such an arrangement may be referred to as a two-dimensional matrix of nanopore structures, or an array of nanopore sensors.

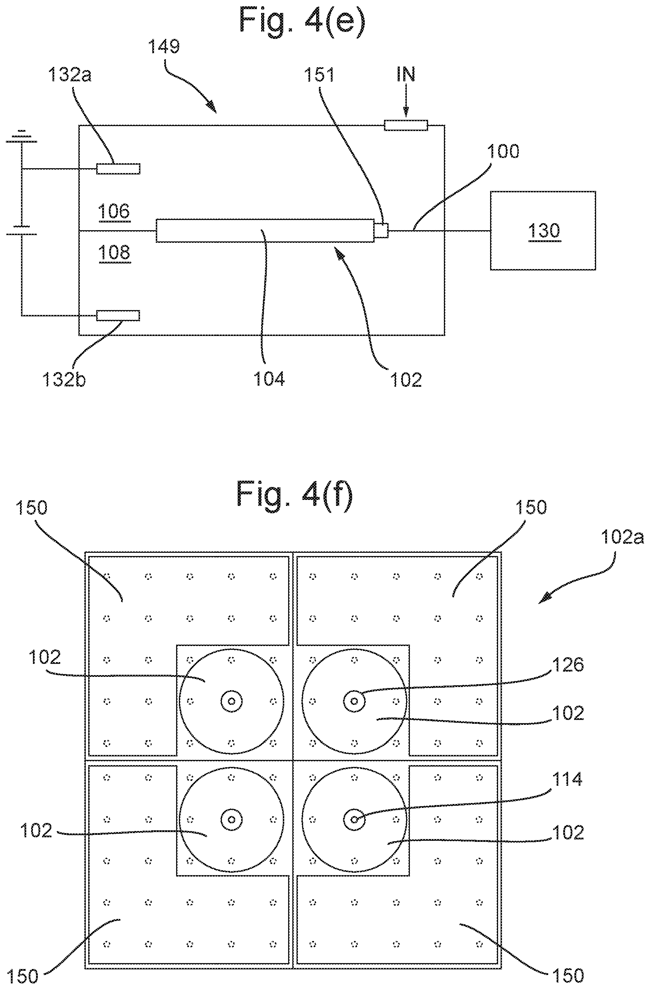

The structure 100, which may take the form of a sheet, incorporates the array of nanopore structures 104 (n.b. only one nanopore sensor 102 of the array is shown) and can be configured within a device or device for analysing an analyte, as shown in Fig. 4(e).

The structure 100 separates the analyte reservoir 106 for receiving an analyte and an outlet chamber 108. The structure 100 has a nanopore layer 110 configured upon a base layer 112, which together forms at least a portion of the structure 100 having a plurality of nanopore sensors 102. Each nanopore sensor 102 in the array 104 has a passage 114, or fluidic passage, configured to extend through the nanopore layer 110 and base layer 112 of the array 104 for connecting the analyte reservoir 106 and outlet chamber 108. The analyte reservoir 106 may also be known as an analyte chamber, a sample chamber, a cis, a cis reservoir, or a first fluidic reservoir. The outlet chamber may also be known as a trans, a trans reservoir, or a second fluidic reservoir.

The nanopore layer 110 of each nanopore sensor 102 is provided with a nanopore 116 in a membrane 118 supported by the nanopore layer 110. Alternatively, the nanopore 116 can be a so-called solid state nanopore, namely a nanometer sized through-hole provided in a solid-state support layer. Further alternatively, the nanopore 116 can be a so-called hybrid nanopore, namely a biological nanopore provided in an aperture in a solid-state membrane. Either way, the nanopore 116 is provided in a membrane 118 *proximal* the first end 120, or pore end, of the passage 114 (e.g. at the top of the sensor as shown).

The base layer 112 has a channel 122 proximal a second end 124, or channel end, being an opposite end of the passage 114 to the first end 120 (e.g. at the bottom of the nanopore sensor 102 as shown). The passage 114 extends through the nanopore structure 100 connecting one side to the other. The channel 122 forms part of the passage 114. The channel 122 is structurally and geometrically configured to function as a fluidic resistor. This can be achieved by defining the aspect ratio of the channel 122. Additionally, or alternatively other techniques for implementing fluidic resistance in the channel 122 can be used.

The fluidic resistance of the channel 122 can be varied by varying its dimensions, in particular its aspect ratio and by varying the ionic concentrations of the fluids in the analyte reservoir 106 and the outlet chamber 108. For example, the channel 122 can be configured with a high aspect ratio to increase the resistance. Additionally, or alternatively, the fluid in the channel 122 can have a lower ionic concentration compared to the fluid in the analyte reservoir 106 and outlet chamber 108 to increase the channel's resistance. Maintaining a higher ionic concentration in the analyte reservoir 106 and the outlet chamber 108 improves the signal to noise ratio.

In some embodiments, the aspect ratio may, for example, be between about 100:1 to about 2000:1 , which is a ratio of channel length to channel diameter or largest transverse dimension.

In some embodiments, the ionic concentration difference may be between about 1:1 to about 2000:1, for example around 1000:1, which is a ratio of ionic concentration in the analyte reservoir 106 and/or outlet chamber 108 to the ionic concentration in the channel 122.

The channel 122 can be configured such that the resistance of the channel 122 and the nanopore 116 are substantially matched, when the passage 114 is occupied by fluid, and relatively high relative to the resistance of fluid in the analyte reservoir 106 and outlet chamber 108 such that the resistance of the analyte reservoir 106 and outlet chamber 108 does not appreciably influence the measurements. In other words, the channel 122 is configured as a fluidic resistor to approximate the resistance of the nanopore 116. This means that the resistance of other circuit elements such as the fluid in the analyte reservoir 106 and outlet chamber 108 has less significance and does not require compensation to account for it when measurements are taken.

The signal-to-noise ratio may be optimised by selecting the fluidic resistance of the channel 122 to be equal to the resistance of the nanopore 116. However, this is not essential and the fluidic resistance of the channel 122 may be varied from this value to take account of other factors, while still obtaining an acceptable signal-to-noise ratio. An acceptable signal-to-noise ratio may be achieved with the fluidic resistance of the channel 122 being significantly less than the resistance of the nanopore 116, for example with the fluidic resistance of the channel 122 being 10% or less of the resistance of the nanopore 116, for example 2% of the resistance of the nanopore 116 or less. In some embodiments, a lower limit on the fluidic resistance of the channel 122 may be set by the desired signal to noise ratio. In other embodiments, a lower limit on the fluidic resistance of the channel 122 may be set by the threshold for crosstalk between adjacent channels during flicking (as described below). That is, the fluidic resistance of the channel 122 is desirably significantly greater than the resistance from the end of the channel 122 to the electrical transduction element to prevent these resistances forming a voltage divider which applies a fraction of the applied voltage to adjacent channels 122.

Other factors that may be considered in the selection of the fluidic resistance of the channel 122 are as follows.

As the fluidic resistance of the channel 122 increases, the diffusion of ions decreases, causing increased depletion of ions near the pore, and thereby causing a decay of the signal over the timescale of a typical event over which a signal is obtained. In order to increase the limit on read length caused by this effect, the fluidic resistance of the channel 122 may be reduced. In many embodiments, this factor may place an upper limit on the fluidic resistance of the channel 122.

As the channel 122 and the nanopore 116 act as a voltage divider, the voltage across the nanopore 116 is affected by the current flowing through it. As the fluidic resistance of the channel 122 increases, the variation in the voltage across the nanopore 116 increases, which can complicate signal processing. In order to limit this effect, the fluidic resistance of the channel 122 may be reduced.

Channels having lower fluidic resistances are easier to fabricate, and may open up alternative fabrication techniques that improve yield or reduce cost.

Reducing the fluidic resistance of the channel 122 may increase bandwidth or provide leeway for additional capacitance in the passage 114.

Taking into account these factors, the fluidic resistance of the channel 122 may be less than the resistance of the nanopore 116, typically at most 50%, or at most 25% of the resistance of the nanopore 116. In some embodiments, the optimal fluidic resistance of the channel 122 may be around 10% of the resistance of the nanopore 116.

When reducing the ratio of the fluidic resistance of the channel 122 to the resistance of the nanopore 116, the signal to noise ratio does not scale directly with that resistance ratio. For example, in some embodiments when the fluidic resistance of the channel 122 is around 10% of the resistance of the nanopore 116, then the signal to noise ratio is around 30% of its optimal value.

The channel 122 can be formed in a wafer, and after a passage 114 is formed therethrough an oxide layer can be used to reduce the diameter of the passage 114 through the base layer 112, thus enabling the amount of oxidisation to adjust the aspect ratio.

A sensor electrode 126, or sensor element, is disposed between the nanopore 116 and at least a portion of the channel 122. The sensor electrode 126 forms the electrical transduction element in this example. More generally, the sensor electrode 126 could be adapted to form an electrical transduction element of any of the various types disclosed in WO2016/127007.

The sensor electrode 126 is exposed, at least in part, to the passage 114 in the nanopore sensor 102, and configured with a connection 128 for measuring electrical potential of the fluid at the location of the sensor electrode 126 when a fluid is provided in the passage 114. Together with the nanopore layer 110 and base layer 112, the sensor electrode 126 defines the walls of the passage 114. The connection 128 can be a wire-bond to a separate electronic circuit 130, said electronic circuit 130 configured to analyse signals obtained from the sensor electrode 126.

The analyte reservoir 106 can function as a first fluidic reservoir, while the outlet chamber 108 can function as a second fluidic reservoir. The structure 100 can separate, at least in part, the analyte reservoir 106 and the outlet chamber 108 and the passage 114 of a sensor 102 connects the analyte reservoir 106 to the outlet chamber 108.

In use, the passage 114 of each nanopore sensor 102 is occupied by a fluid. Further, drive electrodes 132 in the analyte reservoir 106 and outlet chamber 108 comprise at least one respective cis electrode 132a, also known as an analyte electrode, and at least one respective trans electrode 132b, also known as an outlet electrode, configured to impose an electrical potential difference across the passages 114 of the nanopore sensors 102 in the array 104 between the analyte reservoir 106 and the outlet chamber 108.

The structure 100 can be substantially planar. The array 104 can be substantially planar. Non-planar configurations are envisaged by the inventors but not described herein. The nanopore sensors 102 in the array 104 have a cis-surface 134 of the nanopore layer 110 arranged facing the analyte reservoir 106 and defining a cis-plane 136, and a trans-surface 138 of the base layer 112 for facing the outlet chamber 108 and defining a trans-plane 140. This cis-plane 136 and trans-plane 140 are indicated by the hashed line in Figs. 2, 3(b) and 7(b). The sensor electrode 126 is embedded within the structure 100 between the cis-plane 136 and the trans-plane 140. The nanopore 116 can lie substantially on the cis-plane 136 at the first end 120 of the passage 114 while the second end 124 of the passage 114 can lie substantially on the trans-plane 140.

As shown in the assembly of Fig. 2, the sensor electrode 126 can, at least in part, be embedded between in the structure 100 between the nanopore layer 110 and the base layer 112. In other words, the sensor electrode 126 is sandwiched or laminated between the nanopore layer 110 and the base layer 112.

The nanopore layer 110 has a well 142 formed at the first end 120 of the passage 114. In the example of Fig. 2 the nanopore 116 is configured at the first end 120 of the passage 114, on one side of the well 142, substantially on the cis-plane 136. The sensor electrode 126 is configured on the opposite side of the well 142 to the nanopore 116, as shown. The well 142 is shown as a cup-shaped recess with a membrane 118, shown in cross-section, across its rim. The well 142 is configured to receive an analyte that has passed through a nanopore 116. Note that the well 142 is fluidly connected to the channel 122 via a well aperture 142a, which can be described as a well outlet. The well aperture 142a functions to enable the analyte chamber to be fluidly connected to the outlet chamber. The well aperture 142a does not function as a nanopore. In some implementations, the well aperture 142a is configured to enable an analyte to pass therethrough uninhibited i.e. without influencing movement of the analyte from the analyte reservoir 106 to the outlet chamber 108.

Although the well aperture 142a provides a fluid connection between the analyte reservoir 106 and outlet chamber 108 an analyte that has passed through the nanopore 116 can remain in the well 142. The well 142 and channel 122 can be considered part of the outlet chamber 108. In the example shown in Fig. 2, the well aperture 142a is centrally located at the base of the well 142 within the sensor electrode 126.

The well 142, and more generally the nanopore layer 110, is configured for supporting a membrane 118 such as a polymer membrane or lipid bilayer, which may be referred to as a fluid membrane. The nanopore layer 110 can be fabricated separately from the base layer 112. The nanopore layer 110 can be formed from a different material from the base layer 112. The nanopore layer 110 can be formed from at least one of: a photolithographically prepared material; a moulded polymer; or a laser etched plastic.

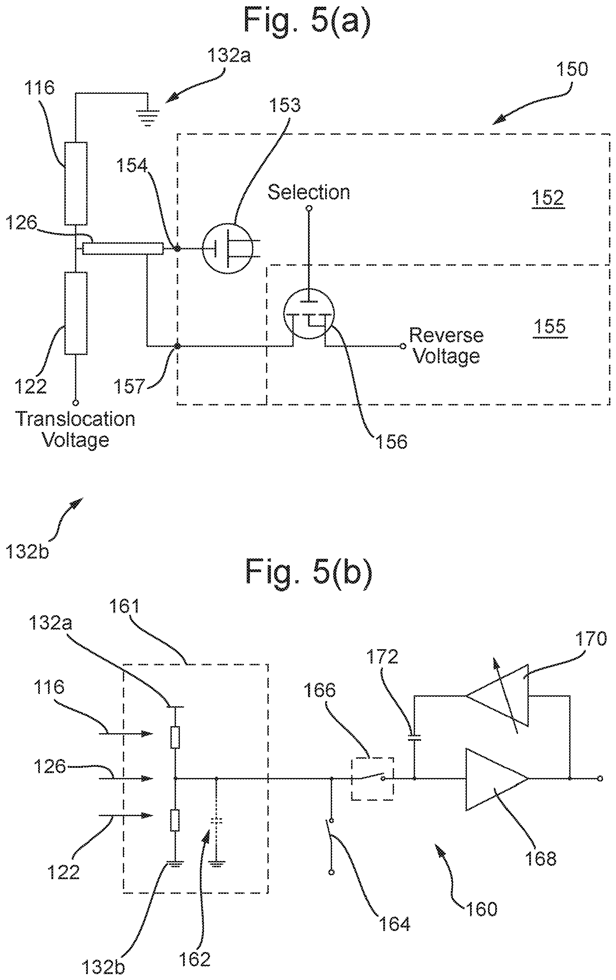

In some embodiments, the sensor electrode 126 is directly connectable to the base or gate of a sensing transistor 153 (shown in Fig. 5(a) and described further below) for measuring electrical potential of the fluid at the location of the sensor electrode 126 when a fluid is provided in the passage 114. In some cases, the sensor electrode 126 can be connected to an edge-connector or wire-bond, optionally by a conductive via and/or interconnect, to an electronic circuit 130, which is an example of a measurement circuit, off-structure. The sensing transistor 153 can be a field effect transistor (FET) and the configuration of the sensing transistor 153 and its optional integration in to the structure 100 is described in an example below. In some embodiments, the sensing transistor 153 (not shown) can be located in the electronic circuit 130.

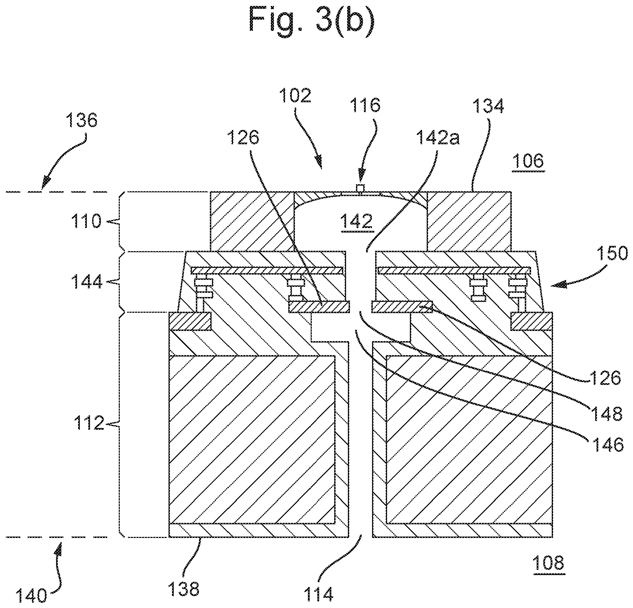

The nanopore sensor 102 shown in Fig. 2 is an example in which the sensor electrode 126 can be formed upon the base layer 112. While the sensor electrode 126 of Fig. 2 can be formed on the base layer 112 directly it can, alternatively, be formed separately upon a sense layer 144 , as depicted in Fig. 3(a) and Fig. 3(b). After forming the sensor electrode 126 on a sense layer 144, the sense layer 144 is then sandwiched between the nanopore layer 110 and base layer 112, resulting in the structure shown in Fig. 3(a).

The sense layer 144 can be fabricated in a similar manner to the base layer 112, wherein a wafer has passages 114 formed therethrough, substantially perpendicularly to the surfaces of the wafer. Alternatively, the wafer can be post-processed to open up the passage 114. The passages 114 and/or channels 122 can be formed using techniques such as photolithography or deep reactive-ion etching (DRIE) or combinations thereof. The wafer can be enclosed by an oxide layer. The wafer can be a CMOS wafer. The sensor electrode 126 can be formed on the sense layer 144 around the passages 114 on one side of the sense layer 144. The passages 114 through the sense layer 144, and the sensor electrode 126 formed around these passages 114, are arranged to have a pitch or layout that results in alignment with channels 122 on the base layer 112. When secured together, the passages 114 of the sense layer 144 are aligned with the channels 122 of the base layer 112.

By way of example, the nanopore layer 110 may be made of polymer, which can be moulded or lithographically etched; the base layer 112 may be formed of a silicon wafer; and/or the sense layer 144 may be a CMOS wafer.

The sense layer 144 can be aligned and bonded to the base layer 112 in one of two orientations. In one orientation (not shown) the sensor electrode 126 remains fully exposed after bonding - that is to say that the sensor electrode 126: is not sandwiched between the sense layer 144; is distal from the base layer 112 after the sense layer 144 is secured to the base layer 112; and is subsequently sandwiched between the sense layer 144 and the nanopore layer 110. In the other orientation, as shown in Fig. 3(a), the sensor electrode 126 is formed on top of a sense layer 144 which is then inverted before bonding to the base layer 112 such that the sensor electrode 126 faces down, as viewed, and is sandwiched between the sense layer 144 and the base layer 112. Prior to bonding in this configuration, a section of the oxide layer on the base layer 112 around the channel 122 can be etched away or otherwise removed to create a cavity 146 such that there is an increased area of the sensor electrode 126 exposed to the passage 114 after bonding. The area of exposed sensor electrode 126 can be maximised to increase contact with a fluid in the passage 114.

The wells 142 of the nanopore layer 110 are aligned with the passages 114 and sensor electrodes 126 and bonded to the sense layer 144 with the sensor electrodes 126 sandwiched therebetween. Looking at Fig. 3(b), and noting that the sense layer 144 is fabricated from the bottom upwards, the last stage is the application of the sensor electrode 126 on top. When assembled, the sense layer 144 is flipped over such that the sensor electrode 126 that was on top is now facing downwards, as shown in Fig. 3(b). The cavity 146 etched out of the base layer 112 oxide layer (the grey part) means that the sensor electrode 126 is sufficiently exposed

The sensor electrode 126 remains exposed, at least in part, to the passage 114 and configured with a connection for measuring electrical potential of the fluid at the location of the sensor electrode 126 at the nanopore 116 when a fluid is provided in the passage 114. Arrangements of the sensor electrode 126 - which minimise its surface area openly facing one of the analyte reservoir 106 or outlet chamber 108 (e.g. the arrangements of Fig. 2 or Fig. 3(a)) - function to limit exposure to the analyte reservoir 106 or outlet chamber 108 to inhibit contamination of the surface of the sensor electrode 126. One such example is shown in Fig. 3(a) that shows the sensor electrode 126 substantially partially enclosed in the passage 114. Before population with a fluid, or during the formation of an amphiphilic membrane for supporting a biological nanopore, the surface of the sensor electrode 126 can be exposed to fluids that could contaminate the surface of the sensor electrode 126, thus if there is a contamination risk then it can be mitigated.

In one configuration, at least a portion of the sensor electrode 126 can be arranged to face away from the well 142 toward the channel 122, as shown in Fig. 3(a). An exposed portion of the sensor electrode 126 provides a connection to a fluid in the passage 114 for sensing fluctuations in the fluidic electrical potential at the sensor electrode 126 when an analyte passes through the nanopore 116. The sensor electrode 126 may also have an embedded portion embedded within the structure 100. The sensor electrode 126 can also have a connection 128, such as a wire-bond, for connection to an electronic circuit 130, such as a measurement circuit or control circuit, which can be separate from the structure 100 as shown in Fig. 3(a).

In each of the examples, the sensor electrode 126 can be configured in various configurations for exposure to a fluid within the passage 114 and can, at least one of: cover, at least in part, a wall of the passage 114; cover, in cross-section, a portion of a wall of the passage 114; form an annulus around the passage 114; form, at least in part, the surface of the base layer 112 or the sense layer 144 around the passage 114 and have an exposed portion arranged to face the analyte reservoir 106; form, at least in part, the surface of the sense layer 144 around the passage 114 and have an exposed portion arranged to face the outlet chamber 108. In particular, a cavity 146 can be formed in the passage 114 to create a region that enables the area of sensor electrode 126 exposure to be increased and come in to contact with an increased amount of fluid. The cavity 146 is formed by recesses formed in the base layer 112 and/or sense layer 144 before the base layer 112 and sense layer 144 are aligned and connected.

While the sensor electrode 126 can have a minimal degree of exposure to the fluid in a well, such as in the form of a nanowire, the inventors have proposed the examples herein to optimise performance of the nanopore sensor 102 and improve manufacturability.

As shown in Fig. 2 and 3(a) the sensor electrode 126 is substantially planar and shaped to accommodate the passage 114. In other words, the sensor electrode 126 is configured to enable uninhibited fluid communication between the analyte reservoir 106 and outlet chamber 108, which can be achieved by either (i) shaping the sensor electrode 126 to extend around the passage 114 or well aperture 142a, and/or (ii) forming a sensor aperture 148 in the sensor electrode 126.

The footprint of the exposed portion of the sensor electrode 126 can be any shape. The well 142 of Fig. 2 and the cavity 146 of Fig. 3(a) can be cylindrical such that the floor of the well 142 is circular, or a planar surface of the cavity 146 is curved. These configurations result in the exposed portion of the sensor electrode 126 being circular or disc-shaped. In the examples shown a sensor aperture 148 is provided such that the footprint of the exposed portion is shaped like an annulus. The exposed area of the sensor electrode 126 can be maximised, which can mean covering at least one face or surface of the well 142 and/or cavity 146.

The sensor electrode 126 and the sensor aperture 148 are shown as circular but could have other shapes. In some embodiments having circular shapes, the ratio of the radius of the exposed portion of the sensor electrode 126 to the radius of the sensor aperture 148 can be in a range from 100:1 to 10:1 or about 2:1. In the case of non-circular shapes, the ratio of the square roots of the areas may have the same values.

Alternatively, the area of exposed portion of the sensor electrode 126 can be expressed in relation to the ratio between the area or footprint as viewed of the sensor aperture 148 can be about 1:1, or about 10:1 or about 100:1.

By way of example, the sensor electrode 126 may have a diameter (or maximum dimension) in a range from 10µm to 50µm and the sensor aperture 148 may have a diameter (or maximum dimension) in a range upwards from 0.5µm. The sensor aperture 148 does not function as a sensor so its size does not have an upper limit within the bound that it is desirable to minimise the restriction of the remaining area of the sensor electrode 126.

The sensor electrode 126 may be formed from a suitable conductive material, for example platinum or gold.

While Figs. 2 and 3(a) have a sensor electrode 126 having a connection 128 to a separate electronic circuit 130, Fig. 3(b) illustrates that the structure 100 and array 104 can accommodate an integrated circuit 150. The integrated circuit 150 can incorporate one or more of the functions of the electronic circuit 130. In other words, various functions, such as sensing, amplifying, controlling, filtering, reading out etc which can be implemented on the separate electronic circuit 130 can be implemented, alternatively, on the integrated circuit 150. The integrated circuit 150 can be formed on a separate layer or wafer and subsequently connected to the sense layer 144 having the sensor electrode 126 thereon. The inventors envisage, however, that the integrated circuit 150 is fabricated within the sense layer 144 together with the sensor electrode 126. An integrated circuit 150 can be provided for each nanopore sensor 102.

In some approaches, after fabrication of the sense layer 144 having the integrated circuit 150 and sensor electrode 126 formed and exposed on one side, the sensing structure is flipped and bonded to the base layer in the same way as it was in relation to Fig. 3(a). Connections 128 (not shown in Fig. 3(b) connect the integrated circuit 150 with a connector 151 for sending signals or data produced by the integrated circuit 150 off the structure. The connections 128 can be connected to a connector 151 as shown in Fig. 4(e), although other configurations are implementable. With the sense layer 144 connected to the base layer 112 the nanopore layer 110 can be formed thereon such that the sense layer 144 is sandwiched between the nanopore layer 110 and the base layer 112. As before with Fig. 3(a), when bonded together, the passages of the sense layer 144 align with the channels of the base layer 112 and the well 142 of the nanopore layer 142 form a portion of the passage 114.

In use, the electronic circuit 130 and/or integrated circuit 150 is configured to detect resistance changes at the nanopore 116 when an analyte, such as a polymer, passes through the nanopore 116, said resistance change detected through the fluid in the nanopore sensor 102 (strictly speaking a measure of resistance being detected as a voltage over the effective voltage divider, as described above). In an array 104 the integrated circuit 150 of each nanopore sensor 102 can be communicably addressable. In light of parasitics, noise from communications and background noise the detected resistance can be difficult to read directly using an off-board processor. To provide a processor with a better signal, i.e. a cleaner reduced noise signal, the integrated circuit 150 can be configured to locally transform or modify or otherwise process signals derived from the detection of a polynucleotide or other analyte passing through the nanopore 116. The integrated circuit 150 can be configured to at least one of: amplify signals, such as amplifying a voltage level of the signal; filter the signal, for example to remove noise; sample the signal; digitise the signal using an analogue to digital converter (ADC) implemented in the electronic circuit 130.

The integrated circuit 150 can be formed within a nanopore sensor 102 footprint within the array 104 of the structure 100.

By way of example, each nanopore sensor 102 of the array 104 can be contained within a pixel 101 which is a footprint of the nanopore sensor 102, as viewed in Fig. 4(a), which can be considered to represent a schematic plan view of a nanopore sensor 102 shown in Fig. 3(b). As viewed, each pixel 101 accommodates a nanopore sensor 102, an sensor electrode 126 and integrated circuit 150. The sensor electrode 126 and integrated circuit 150 can be arranged to inhibit noise interference created by the integrated circuit 150 from being detected by the sensor electrode 126. For example, the integrated circuit 150 may be separated from the nanopore sensor 102, as depicted in Fig. 4(a). This separation can be implemented by configuring the integrated circuit 150 to be located outside the pixel 101, as viewed.

This separation may also simplify the manufacturing process. Alternatively, the integrated circuit 150 can be distanced from the sensor electrode 126 (i.e. the distance between the sensor electrode 126 and the integrated circuit 150 in the depth direction, or thickness of the structure 100, is increased to minimise noise interference. Note that the depth direction of Fig. 4(a) is in a direction in to and out of the page, as viewed.

In the example shown, the pixel 101 is square and has a side length of 20µm, but in other examples may be in a range from 10µm to 50µm.

By way of example, the integrated circuit 150 occupies about three-quarters of the pixel 101, while the remaining quarter is occupied by the sensor electrode 126 which has a diameter of 10 µm in the example shown.

Other arrangements are envisaged. In some embodiments, the sensor electrode 126 may be larger than the example shown in Fig. 4(a), for example covering almost all of the area of the nanopore sensor 102. In some embodiments, the sensor electrode 126 may be have other shapes covering more area, for example square or rectangular. The sensor electrode 126 may have dimensions of up to 50µm, in which case it may have an area of up to 250µm², depending on its shape.

For packaging efficiency, the pixel 101 can be tessellated, and, for example, the tessellation can be hexagonal.

Each nanopore sensor 102 has a passage 114, although during fabrication of the base layer 112 more channels 122 could be created in the base layer 112 than are needed, depending on the method of fabrication. Some methods of fabrication such as reactive ion etching can etch a single channel 122 for each pixel 101. Some other method such as photo assisted electrochemical etching requires a high-density array of channels 122 to be etched at the same time to maintain the geometry of those channels 122 - in this case unused channels 122 in the base layer 112 are blocked during fabrication of the array 104 such that only one channel 122 and one passage 114 are provided per pixel 101. The density of the channels 122 formed in the base layer 112 can vary. Fig. 4(b) shows, by way of comparison, a cross-section of a nanopore sensor 102 having a lower density of blocked channels 122a than that shown in Fig. 4(a). The channels 122, as shown in Fig. 4(b) can be blocked prior to the sense layer 144 being added to the base layer 112, or may be blocked by a substrate of the sense layer 144. It is to be noted that Fig. 4(b) is shown with portion of two nanopore sensor 102, each with its own passage 114, and has not yet had a nanopore layer 110 added upon the sense layer 144.

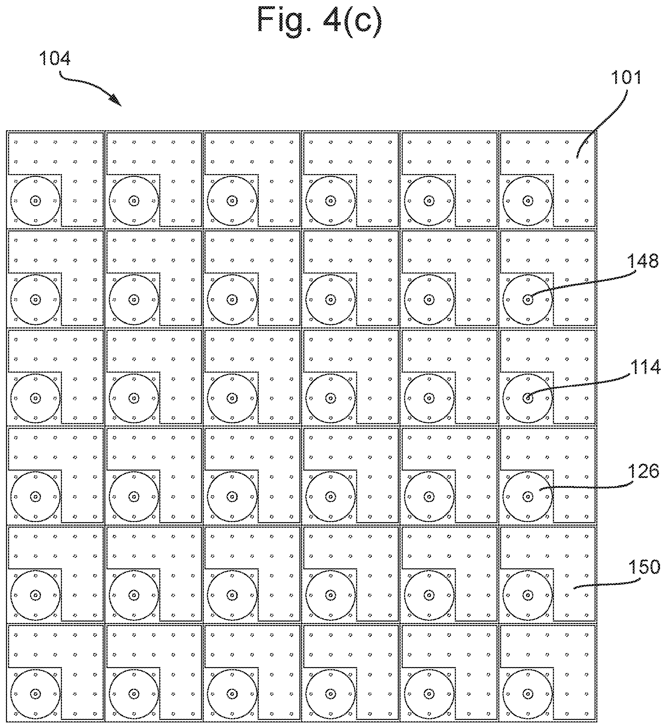

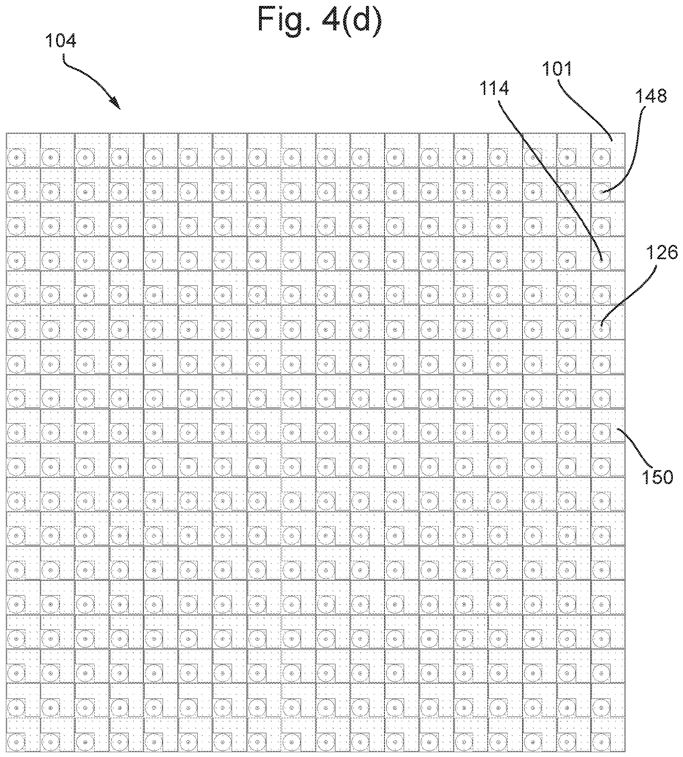

Fig. 4(c) shows the nanopore sensor 102 pixel 101 of Fig. 4(a) arranged in a 6x6 layout providing an array 104 of 36 nanopore sensors 102, while Fig. 4(d) has an 18x18 array having 324 nanopore sensors 102. The array size can be 1000x1000, providing 1,000,000 nanopore sensors 102. In the present example, an array of one million nanopore sensors 102 of the type shown in Fig. 4(a) would have a footprint of 4cm², however nanopore sensors 102 having pixels 101 as small as 5µm can bring the footprint of a one million nanopore sensor array 104 down to around 25mm². The array size can be 100,000. The array 104 may comprise any number of nanopore sensors 102 between 1000 and 10 million nanopore sensors 102.

Fig. 4(e) shows an array 104 having nanopore sensors 102 as described herein arranged in a structure 100 provided in a device 149 for receiving and analysing an analyte of polymer such as nucleic acid. The device 149 may also be known as a sensor device or measurement system. The array 104 can be a sub-component of the device 149. The array 104 can be a disposable component and replacable. Additionally, or alternatively, the nanopore layer 110 of the array 104 can be a disposable component and replacable. The device 149 can include an electronic circuit 130 as described above.

In some embodiments, processing of the signals measured by a nanopore sensor 102 can be performed by the electronic circuit 130. The integrated circuit 150 can perform pre-processing prior to further analysis by the electronic circuit 130 of the device 149.

In some embodiments, the device 149 houses the structure 100 to separate and define the analyte reservoir 106 and outlet chamber 108. While often referred to, respectively, as the cis and the trans, the analyte can flow from the analyte reservoir 106 to the outlet chamber 108. The array 104 has a plurality of nanopore sensors 102, each with a passage 114 therethrough, to fluidly connect the analyte reservoir 106 and outlet chamber 108. By way of example, the drive electrodes 132 in the analyte reservoir 106 and outlet chamber 108 can impose an electrical potential difference across the passage 114, between the analyte reservoir 106 and outlet chamber 108, to induce an analyte to flow from the analyte reservoir 106 to the outlet chamber 108. The drive electrodes 132 can be configured such that the potential difference is substantially the same across all the nanopore sensors 102.

Additionally, or alternatively, the device 149 can be configured to induce an analyte from the analyte reservoir 106 to the outlet chamber 108 using other techniques. As an analyte passes through a nanopore 116 the fluctuation in electrical potential caused by changes in ion current flow is detected by the sensor electrode 126.

The sensor electrode 126 can function as, or be connected directly to, the base or gate of a sensing transistor 153 (as shown in Fig. 5(a) and described further below), which can be a field effect transistor (FET) device for example. The sensing transistor 153 outputs a signal that can be processed by the integrated circuit 150 of each nanopore sensor 102, which can then be addressed in a row-column type manner. For example, the voltage at the drain of the sensing transistor 153 may depend upon the electrical potential sensed by the sensor electrode 126, and the voltage at the drain can be read out, along with other drain voltages on other nanopore sensors 102 in an array 104, in a row-column manner. The processed signal can then be analysed further - off the array 104 - to determine one or more properties of the analyte.

In the examples above each pixel 101 has its own integrated circuit 150, but an integrated circuit 150 can be configured to serve a plurality of nanopore sensors 102. In Fig. 4(f), four nanopore sensors 102 are shown as a sensor module 102a (which may be part of a larger array of nanopore sensors 102), wherein the integrated circuit 150 is common to four centrally located sensor electrodes 126, as shown. Other configurations are feasible. In such module configurations the information or data obtained from each individual nanopore sensor 102 is addressable for control and/or retrieval of information. While the examples above have a dedicated integrated circuit 150 for each nanopore sensor 102, combining nanopore sensors 102 in a sensor module 102a enables the efficiency of the layout to be improved. Efficiency improvements can, for example, be achieved because a common filter is used for each of the nanopores 116 within the sensor module 102a. This is possible if the integrated circuit 150 switches or multiplexes between the individual nanopore sensors 102 in turn. By sharing functions between the nanopore sensors 102 either the footprint of the integrated circuit 150 can be reduced or, alternatively, more functions can be accommodated.

Fig. 5(a) is a schematic representation of the immediate connections to the sensor electrode 126 with each nanopore sensor 102 in the array 104. The cis electrode 132a can be connected to ground while a translocation voltage is applied to trans electrode 132b. The resistance of the nanopore 116 and the resistance of the channel 122, which is configured to function as a fluidic resistor, dominate the circuit between the cis electrode 132a and trans electrode 132b via each passage 114 of each nanopore sensor 102. In this way the circuit behaves like a voltage divider having two resistors of similar value. The nanopore resistance and resistance of the channel 122 or fluidic resistor, are approximately the same such that an electrode positioned therebetween is optimally positioned to detect changes in the nanopore resistance caused by an analyte passing therethrough. The sensor electrode 126 resides, as described above, in the region of each nanopore 116. The sensor electrode 126 can lie between the nanopore 116 and the channel 122. The effective impedance of the nanopore 116 and the channel 122 are much larger than the bulk fluidic resistance of the analyte reservoir 106 and outlet chamber 108 - this means that Fig. 5(a) can be used to model the circuit between the cis electrode 132a and trans electrode 132b.

The circuitry includes a sensing circuit 152 which measures fluidic electrical potential at the sensor electrode 126 of the nanopore sensor 102 for taking measurements from the nanopore 116. The sensing circuit 152 may include, for example a sensing transistor 153 which may be a field effect transistor (FET). In this case, the sensor electrode 126 may be connected to the base of the sensing transistor 153. The sensing circuit 152 may reside, at least in part, in the integrated circuit 150. Thus, the sensor electrode 126 may be connected to a sensor terminal 154 of the sensing circuit 152, as shown in Fig. 5(a).

Optionally, the sensing circuit 152 may include a control circuit 155 which applies a signal to the sensor electrode 126 to alter an electrical potential difference across the nanopore 116 imposed by the drive electrodes 132 in response to a control signal. The control circuit 155 may include, for example a control terminal 156 which may be a field effect transistor (FET). In this case, the sensor electrode 126 may be connected to the drain of the control terminal 156. The control circuit 155 can reside, at least in part, in the integrated circuit 150. Thus, the sensor electrode 126 may be connected to a control terminal 156 of the control circuit 155, as shown in Fig. 5(a) for application of the control signal.

The application of the control signal enables an alteration of the potential difference imposed across the individual nanopore 116 by altering the potential difference between the control connection of the control circuit 155 and the cis electrode 132a and/or the trans electrode 132b. The signal applied to the sensor electrode 126 can be a reverse-voltage that induces the charged analyte, such as a species, to change the direction in which it is moving through the passage 114.

In some cases, the voltage applied can be an alternating current voltage, although other voltage waveforms (e.g., ramp, step, impulse, DC) may alternatively be applied.

The integrated circuit 150 of Fig. 5(a) enables a common electrode to be configured for each of the analyte reservoir 106 and outlet chamber 108, while each nanopore sensor 102 can operate to detect an interruption to ion current flow across the passage 114 by detecting variations in electrical potential caused by a variation in nanopore resistance. Furthermore, the integrated circuit 150 enables each nanopore sensor 102 within the array 104 to be individually controlled to enable the sensor electrode 126 to either detect an analyte passing through the nanopore 116 through, for example, a connection with a sensing transistor 153 or control the flow of a charged analyte, such as a species, in the passage 114 of individual nanopore sensors 102 in the array 104 by adjusting the voltage applied to the sensor electrode 126 using, for example, a control transistor 156. The control of the flow of a charged analyte, such as a species, in the passage 114 of individual nanopore sensors 102 in the array 104 allows for an analyte passing through the nanopore 116, or an analyte blocking the nanopore 116, to be passed back or ejected by a voltage applied by the control transistor 156. This action can be described as "flicking" or "rejecting" and occurs by using a control voltage, such that an analyte passing from one side of the structure 100 through the passage 114 is modified - either stopped, reversed or accelerated. A control voltage can be applied to each nanopore 116, individually, because each nanopore sensor 102 is individually addressable for controlling and sensing. To be clear, the application of a control signal to the sensor electrode 126 in each nanopore sensor 102 means that the voltage near the nanopore 116 at each pixel 101 can be controlled.

The control voltage can be applied to alter the movement of an analyte through the nanopore 116 in response to at least one condition from conditions including: when a blocked nanopore 116 is detected; when the analyte detected is no longer of interest and is to be ejected for the purposes of enabling another sample to be received and measured; and to alter the rate at which an analyte is induced in to or out of the nanopore 116.

An electronic sensor, inevitably, has capacitances, resistances and inductances associated with the path along which the sensor signal travels, which may be referred to as parasitics. These are due to the properties of the materials the sensor is constructed from, the geometry of the sensor, and the methods by which it is feasible to fabricate the sensor. Without any kind of capacitance compensation, these parasitics (most commonly the resistances and capacitances) interact to limit the bandwidth of the signal. In the simplest case, a resistor-capacitor circuit will limit the bandwidth to 1 / (2 π R C).

Fig. 5(b) is an alternative schematic of Fig. 5(a) that illustrates the resistor model 161 of the nanopore 116 and the channel 122 and further includes a compensation circuit 160 connected to the voltage divider. According to some embodiments, a compensation circuit 160 has an inline amplifier 168, with gain G, connected to the output of the sensor electrode 126, which is influenced by the parasitic input capacitance 162, also known as stray capacitance. The output of the inline amplifier 168 has a feedback loop connected to its input, said feedback loop having a feedback amplifier 170, with gain H, and a capacitor C representing compensation capacitance *C_{compensation}.*

A parasitic input capacitance 162 is shown arranged in parallel with the resistor representing the channel 122, which represents parasitic capacitance in at least one of: the membrane 118 in which the nanopore 116 rests; the fluidic walls of the channel 122; the sensor electrode 126; and a trace capacitance associated with a connector or wire-bond. The sensor electrode 126 is, in effect, connected to the mid-point in the voltage divider between the nanopore 116 and channel 122 and connected to the compensation circuit 160. The connection to a reverse or flicking voltage is represented by a flicking switch 164, such as a FET. An optional guard switch 166 is shown implemented between the sensor electrode 126 and the compensation circuit 160. This guard switch 166, which can be implemented using a FET, can function to isolate the compensation circuit 160 and /or any sensing circuitry connected thereto from the flicking voltage applied via the flicking switch 164.

Overall, the compensation circuit 160 mitigates the effects of the total parasitic input capacitance 162 at the input to the sensing circuit 152. Although the parasitic capacitance resides in various elements of the nanopore sensor 102 it can be modelled as shown in Figure 5(b). Without being bound to a particular theory, a total parasitic input capacitance 162 *Cₚ* can be considered as a sum of the parasitic capacitances, as follows. ${C}_{p} = {C}_{membrane} + {C}_{fluidic walls} + {C}_{electrode} + {C}_{trace}$

The rate at which the input capacitance charges is proportional to the current flowing through it. **In** turn, the resistance limits the charging current to a finite value. The compensation circuit 160 functions to supply additional current to charge the input capacitance faster, thus increasing the bandwidth.

The compensation circuit 160 of Fig. 5(b) has an inline amplifier 168, with gain G, connected to the output of the sensor electrode 126, which is influenced by the parasitic input capacitance 162. The output of the inline amplifier 168 has a feedback loop to its input, said feedback loop having a feedback amplifier 170, with gain **H,** and a compensation capacitor C 172 representing compensation capacitance *C_{compensation}.*

In some embodiments, the input voltage is amplified and fed back through the compensation capacitor 172 so as to provide additional current to charge the total parasitic input capacitance 162. An effective input capacitance of this circuit may be expressed as: ${C}_{effective} = {C}_{in} - {C}_{compensation}$ wherein ${C}_{compensation} = C ∗ \left(G∗H-1\right)$

The components of the compensation circuit 160 are configured such that the total parasitic input capacitance *Cₚ* 162 is substantially negated or cancelled. **In** practice, the degree of compensation is limited by dynamic changes in the component values and parameters (e.g. temperature dependence). The compensation circuit 160 can compensate for a range of different parasitic input capacitance values if capacitance C, inline gain G or feedback gain H is made adjustable, hence the feedback amplifier 170 is illustrated as variable in Fig. 5(b). The gain G can be fixed such that the output from the compensation circuit 160 has a consistent gain, therefore either the compensation capacitor C 172 and/or the feedback gain H can be varied.

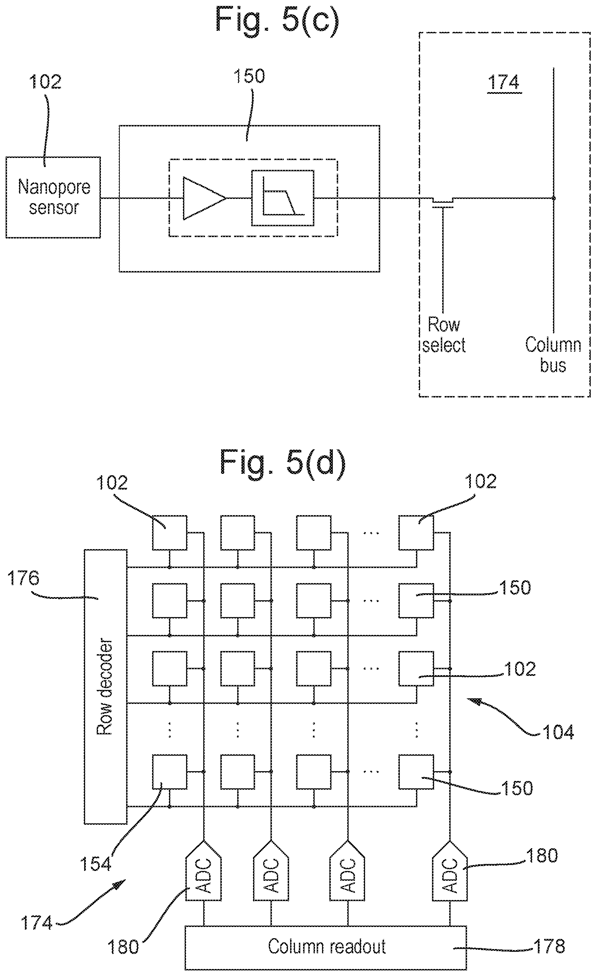

The front-end electronics can reside, at least in part, in the integrated circuit 150, which is figuratively represented in Fig. 5(c). The control circuit 155 and/or compensation circuit 160 can optionally be incorporated within the integrated circuit 150. The integrated circuit 150 or electronic circuit 130 is operable to influence the movement of an analyte in the nanopore 116, such as by flicking, by applying a reverse voltage, and amplifying the signal from the nanopore sensor 102. The integrated circuit 150 or electronic circuit 130 can additionally incorporate further processing of the signal, such as filtering, and may include circuitry to store information locally at the sensor 102, in the case of the integrated circuit 150, for managed communication with an external processor.

Each nanopore sensor 102, such as those shown in Fig. 2 and Fig. 3(a) may be addressable. Fig. 5(c) represents the nanopore sensor 102 of Fig. 3(b) that incorporates the integrated circuit 150 within a pixel 101 shown in Fig. 4(a) and is also addressable via row-selection and column bus. Fig. 5(d) is an example of a row-column readout circuit 174 connected to each nanopore sensor 102 in an array 104, such as the array 104 shown in Fig. 4(d), via a row-selection and column bus connection shown in Fig. 5(c). Each nanopore sensor 102 is connected to a row decoder 176 and column readout 178 via an analogue to digital converter (ADC) 180. The row-column readout circuit 174 can be connected to the integrated circuit 150 of each nanopore sensor 102 or group of nanopore sensors 102 but may be connected directly to the sensor electrode 126 in each nanopore sensor 102 within the array 104.

The examples above describe the sensor electrode 126 being connectable to an integrated circuit 150 and having the option of dual functionality when a control voltage is applied (i.e. the sensor electrode 126 can be used to sense the change of ion flow when an analyte passes through a nanopore 116 and create an electrical potential within the passage 114 and a potential difference across the passage 114 between the cis electrode 132a and/or trans electrode 132b under the control of the control circuit 155). In this case, the sensor electrode 126 is directly connected to a control terminal 156, which is a terminal of the integrated circuit 150, for creating the electrical potential within the passage 114, as shown in Fig. 5(a) and described further below.

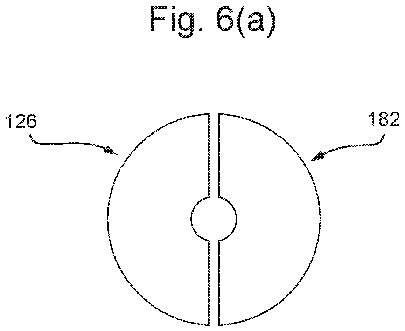

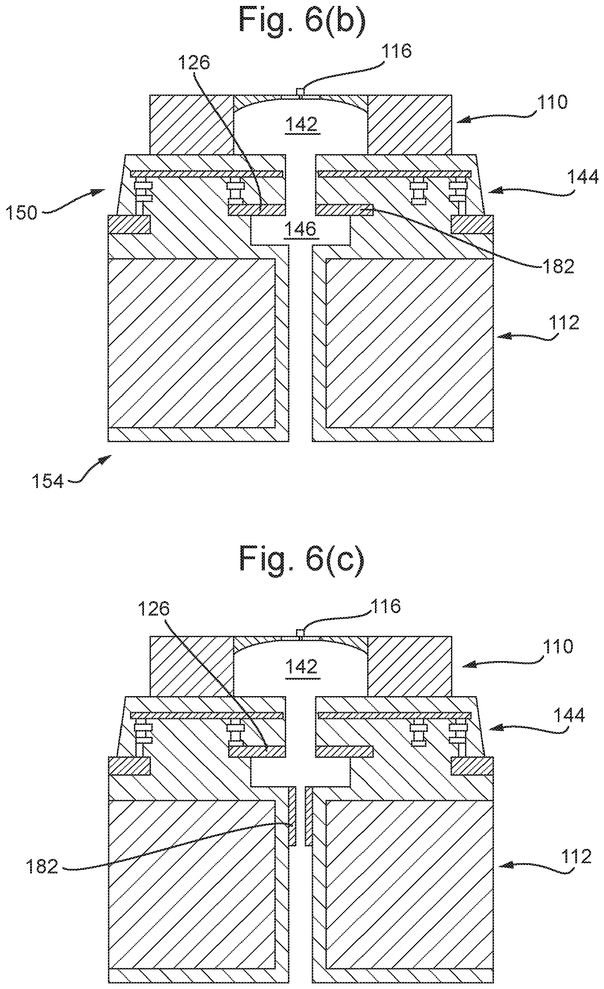

In some implementations, the sensing and control functions in each nanopore sensor 102 may be implemented by separate electrodes, for example as follows. Fig. 6(a) shows a sensor electrode 126 and control electrode 182 arranged like an annulus, while Figs. 6(b) to 6(e) are cross-sectional schematics of nanopore sensors 102 having configurations in which a control electrode 182 is provided in addition to the sensor electrode 126. In this case, the control electrode 182 is connected to the control terminal 156 of the control circuit 155 for creating the electrical potential within the passage 114.

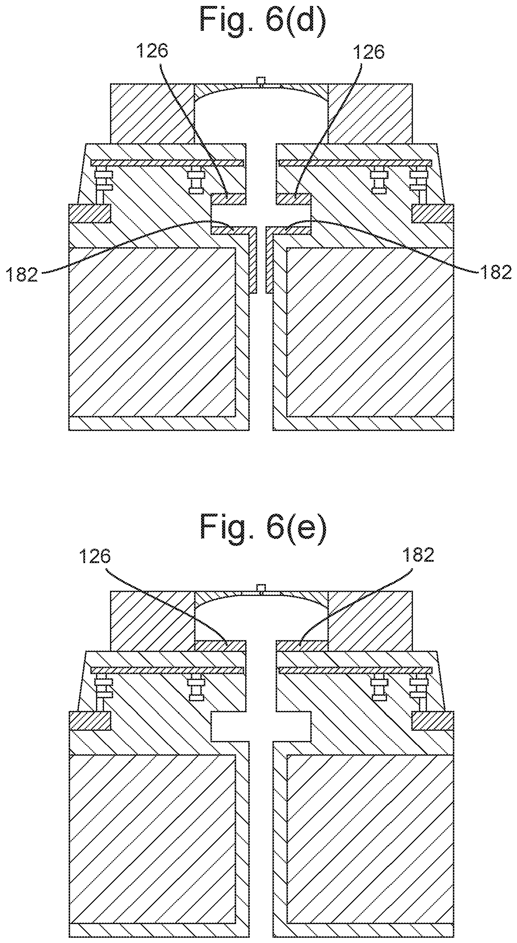

In an example herein, the sensor electrode 126 has been described as an annulus, as illustrated in Fig. 4(a). The sensor electrode 126 could also be implemented by an exposed wire. The sensor electrode 126 could be a nanowire, but can be a larger surface area that occupies, for example, substantially all of the base of a well 142, as shown in Fig. 6(e), or one face of a recess 146. Similarly, a separate control electrode 182 could be a nanowire but can have a large surface area, as shown in Fig. 6(d).

From a manufacturability and cost perspective, a basic implementation of a control electrode 182 is shown in Fig. 6(a), wherein the annulus footprint - suitable for the base of a well 142 - is substantially maintained, while one half of the footprint forms the sensor electrode 126 and the other half, which is physically disconnected or decoupled from the sensor side, forms the control electrode 182. There is no wired or solid-state connection between the sensor electrode 126 and the control electrode 182. The sensor electrode 126 and control electrode 182 are shown having two equally sized semi-circle shapes occupying the footprint. The electrodes can be different sizes, and, for example, the control electrode 182 can have a greater surface area than the sensor electrode 126 to increase the conductivity with the fluid within the passage 114.

Having separate sensor and control electrodes 126, 182 can simplify the integrated circuit 150 because, by being separate, an extra degree of separation is provided, although they will still be connected through a fluid in the passage 114. However, it can be possible to avoid the need of an isolating switch to protect, for example, the compensation circuit 160, which can form part of the sensing circuit 152, from the voltages applied by the control circuit 155. The sensor and control electrodes 126, 182 can be tailored in shape, size and configuration to be optimised for their purpose.

Fig. 6(b) indicates how the sensor electrode 126 of Fig. 3(b) can be divided into a separate sensor electrode 126 and control electrode 182. In this example the sensor and control electrodes 126, 182 extend in the same plane. In an alternative configuration shown in Fig. 6(c) the sensor electrodes 126 reside in the cavity 146 and extend in a plane extending in parallel with the cis-surface 134 and trans-surface 138, while the control electrodes 182 extend in the channel 122 and extend perpendicularly from said cis- and trans-surfaces 134, 138. In Fig. 6(c) the sensor electrode 126 is shaped like an annulus while the control electrode 182 is shaped like a cylinder. In yet another alternative, as shown in Fig. 6(d), the sensor electrodes 126 reside in the cavity 146 and extend in a plane extending in parallel with the cis-surface 134 and trans-surface 138, while the control electrodes 182 extend in the channel 122 and in the cavity 146, thus extending in vertical and horizontal planes, as viewed. Fig. 6(e), which is similar to Fig. 6(b), shows the sensor and control electrodes 126, 182 formed at the base of the well 142, which can offer easier fabrication.

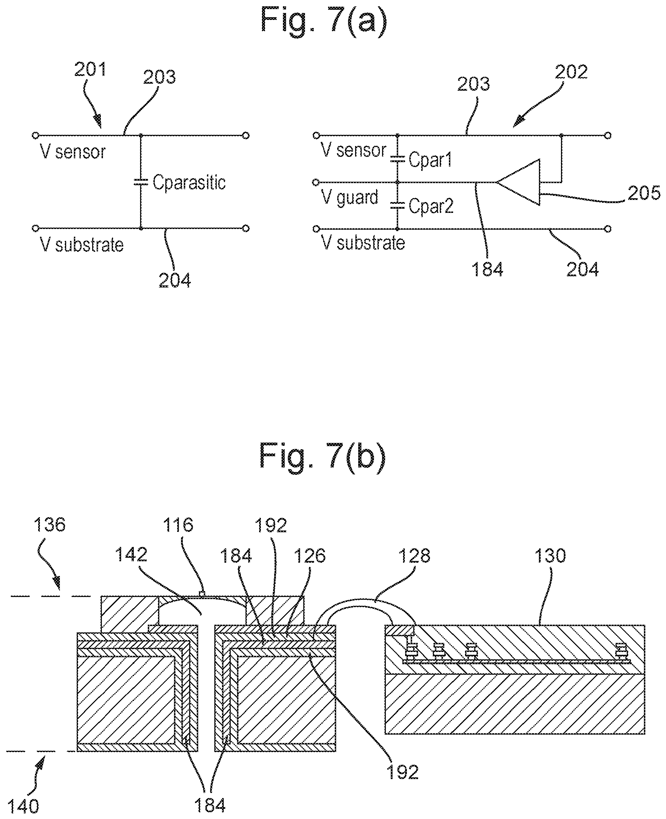

As described above, an electronic sensor inevitably has capacitances, resistances and inductances associated with the path along which the sensor signal travels, which may be referred to as parasitics, which includes parasitic capacitances. Additionally, or alternatively to the compensation circuit 160 described above, the array 104 and nanopore sensors 102 therein can be fabricated with a guard conductor 184 incorporated therein, as shown in Figs. 7(b) to (d), while Fig. 7(a) shows first and second schematic circuits 201 and 202 with and without a guard conductor 184 in order to illustrate how a guard conductor 184 is configured.

In the left-hand, first schematic circuit 201 of Fig. 7(a), the parasitic capacitance *C_{parasitic}* is shown between two conductive elements 203, 204 of the nanopore sensor 102, typically being a conductor, such as the sensor electrode 126, and a conductive substrate of the base layer 112. The first conductive element 203 (e.g. sensor electrode 126) may carry a voltage Vₛₑₙₛₒᵣ and the second conductive element 204 (e.g. the conductive substrate) may carry a different voltage *V_{substrate}.*

Guarding is shown implemented in the right-hand, second schematic circuit 202, wherein guard conductor 184 which is a third conductive element is configured between the first conductive element 203 carrying the signal and the second conductive element 204 such that two parasitic capacitances *Cₚₐᵣ₁, Cₚₐᵣ₂* can be modelled as connected in series. In this second schematic circuit 202, the parasitic capacitance occurs (i) between the first conductive element 203 carrying the signal and the guard conductor 184, i.e. *Cₚₐᵣ₁,* and (ii) between the guard conductor 184 and the second conductive element204, i.e. *Cₚₐᵣ₂.* A buffer 205 (which may be an amplifier) is connected between the first conductive element 203 and the guard conductor 184 to apply a buffered version of the input signal to the guard conductor 184. As a result, there is no voltage difference across the parasitic capacitance *Cₚₐᵣ₁* between the first conductive element 203 and the guard conductor 184.

For a capacitor, the current is given by $I = C \frac{dV}{dt}$

In the right-hand schematic, *V_{guard} = Vₛₑₙₛₒᵣ,* thus $\frac{dV}{dt} = 0$.

No current flows through the capacitor *Cₚₐᵣ₁,* thus the effective capacitance is zero. The capacitance between the guard conductor 184 and the substrate conductors must still be charged, but the buffer 205 is able to supply much more current than the high-impedance sensor input, so it charges much faster.

These conditions are met when *V_{guard}* accurately follows *Vₛₑₙₛₒᵣ,* which depends on the performance of the buffer 205 having sufficient bandwidth to enable the capacitance to be nulled. Precise buffers 205 with bandwidths of several MHz can be implemented.

Fig. 7(b) is analogous to Fig. 2(b) and shows, by way of comparison, a guard conductor 184 extending between oxide layers 192 along the length of the channel 122, vertically as viewed, and continues horizontally, as viewed, along the top of the base layer 112 beneath the sensor electrode 126. Notably, both the sensor electrode 126 and the guard conductor 184 are connected to the separate electronic circuit 130. In this configuration the guard conductor 184 inhibits current flow in the parasitic capacitance between the sensor electrode 126 and the substrate of the base layer 112. A conductive guard can include, at least in part, the guard conductor 184 and an insulating layer, such as the oxide layer 192, that insulates the guard conductor 184 from the conductor being guarded or the conductor being guarded from. The insulating layer is not part of the guard conductor 184 and functions to isolate the guard conductor 184 from surrounding conductors. The insulating layer, therefore, can be an non-conductive component of the structure 100. The guard conductor 184 can be a conductor inserted into the middle of the parasitic capacitances to divide them in two, which is possible because capacitors are by nature insulators, thus the guard conductor 184 is located in an existing insulating layer.

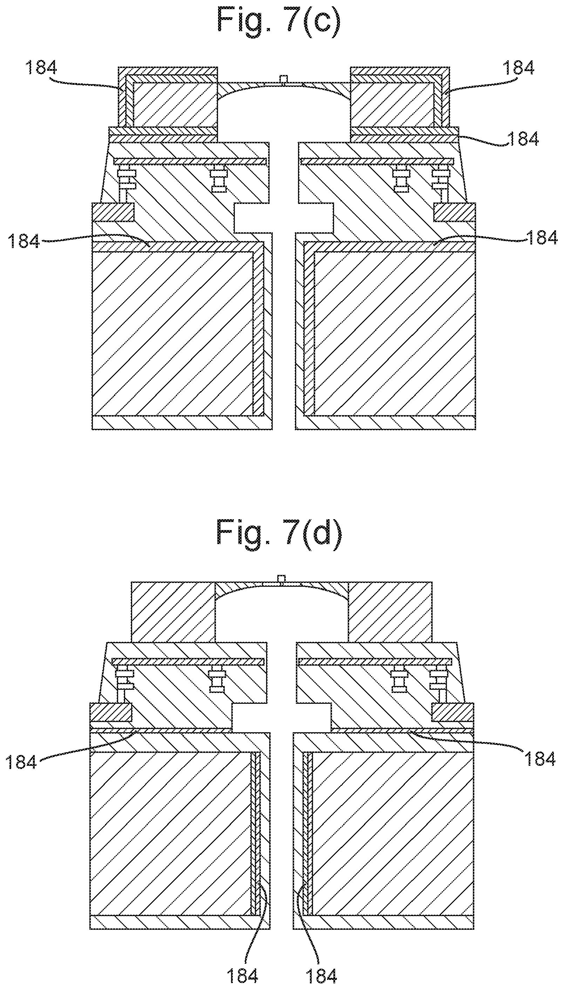

The conductive guard, including an insulating layer, can be configured in a number of different configurations, or combination thereof, comprising at least one of: extending over at least a portion of the nanopore layer 110 for separating the nanopore layer 110 from an analyte in the analyte reservoir 106, as shown in Fig. 7(c), which guards the solution underneath the nanopore 116 from the solution above; extending between the at least a portion of the nanopore layer 110 and the sense layer 144 for separating the sensor electrode 126 and integrated circuit 150 from the solution in the cis 106, as also shown in Fig. 7(c); extending between the base layer 112 and the sense layer 144, at least in part, for separating the sensor electrode 126 and integrated circuit 150 from the base layer 112, as also shown in Fig. 7(c); and a plurality of conductive guards, as shown in Fig. 7(d), wherein a first conductive guard extends between the walls of the channel 122 and the base layer 112 and a second conductive guard extends between the sense layer 144 and the base layer 112.

In light of the teaching herein a skilled person would appreciate that one of the guard arrangements taught herein, or a combination thereof, could be implemented. It will also be appreciated that the guard conductor 184 can be provided in an array 104 of nanopore structures, e.g. the array 104 of Fig. 4(c).

It is to be noted that guard-based capacitance compensation techniques, shown in Figs. 7(a) to 7(d) have the advantage that they generally do not appreciably increase the noise level of the signal. However, such a technique cannot compensate for the membrane capacitance when a potential difference across the membrane 118 is used to drive the analyte being studied through the nanopore 116, but it may be possible to drive the analyte by another means, e.g. pressure. A compensation circuit 160, on the other hand, can compensate for the entire input capacitance, but does so at the expense of added noise. The noise gain of the compensation capacitor 172 increases with frequency. Therefore, the noise in the input signal is scaled by this feedback gain 'G' and adds to the overall noise. This becomes significant at higher frequencies or when compensating for larger input capacitances. The guard conductors 184 shown in Figs. 7(b) to (d) can, therefore, be implemented in the array 104 in any combination and/or in combination with a compensation circuit 160.

The nanopore sensors 102 can be manufactured using a number of different techniques and the functions are taught, by way of example with reference to Fig. 2, which is indicative of the other sensors taught in the application. Although only one of the nanopore sensors 102 of the array 104 is shown in Fig. 2, the fabrication of an array 104 can be understood from the teaching herein. The base layer 112 is formed from a standard silicon (Si) wafer that has channels 122 formed therein to pass from one side of the base layer 112 to the other. Only one channel 122 is shown in Fig. 2, formed through the Si wafer extending substantially perpendicularly to the surfaces of the wafer. In practice, the array 104 has channels 122 formed across the wafer using techniques such as photolithography or deep reactive-ion etching (DRIE) or combinations thereof. At least one channel 122 is formed for each nanopore sensor 102. Techniques such as thermal oxidation can be used to adjust the diameter of the channel 122 to calibrate the aspect ratio, if required. The Si wafer and channels may be embedded in an oxide layer, which may be formed on a silicon wafer, for example.

The example of Fig. 2 schematically shows a portion of the structure 100 having an array 104 of nanopore sensors 102 (only one of which is shown) and is configured to separate an analyte reservoir 106 and outlet chamber 108 having drive electrodes 132 therein. All of the nanopore sensors 102 herein can be located in a structure 100 as shown in Fig. 4(e). The nanopore 116 lies in the passage 114 between the analyte reservoir 106 and the outlet chamber 108, which are fluid filled. The passage 114 is fluid filled such that the analyte reservoir 106 and outlet chamber 108 are fluidically connected. To be clear, the nanopore 116 lies in a path of fluidic communication between the analyte reservoir 106 and outlet chamber 108.

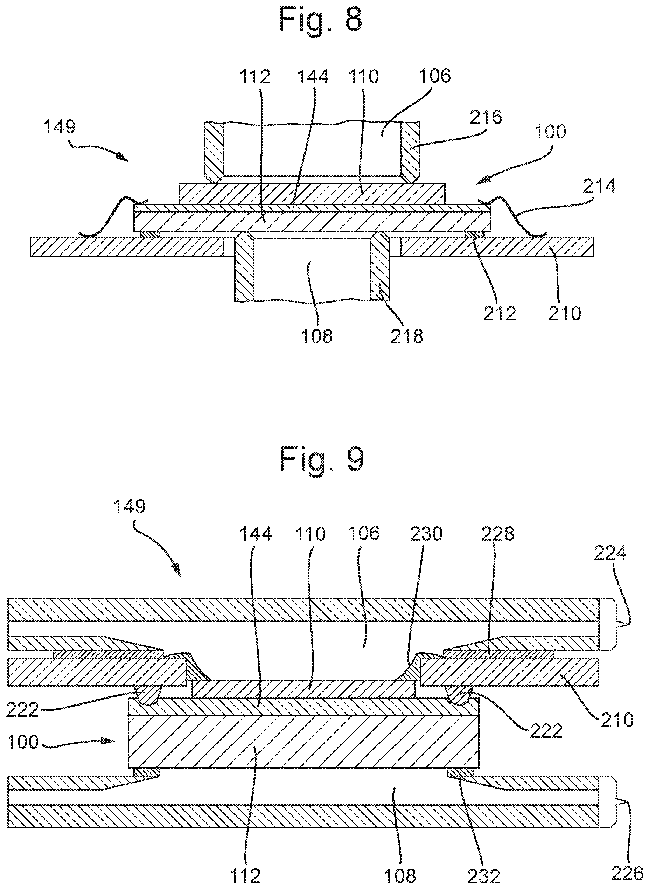

Figs. 8 and 9 show two further examples of a device 149 including a structure 100. In each case, the structure 100 takes the form shown in either Figs. 3(a) or 3(b) including a nanopore layer 110, a sense layer 144 and a base layer 112, as described in detail above (although in each case it could be replaced by a structure 100 taking the form shown in Fig. 2).

In each of the examples of Figs. 8 and 9, the structure 100 separates the analyte reservoir 106 and the outlet chamber 108 and is connected to a printed circuit board 210 but with different configurations as follows.

In the example of Fig. 8, the analyte reservoir 106 and the outlet chamber 108 are each formed by respective gaskets 216, 218 which seal against the nanopore layer 110 and the base layer 112, respectively. The analyte reservoir 106 and the outlet chamber 108 may be open as shown in Fig. 8 or may be closed, for example by respective members extending across the gaskets 216, 218.

In the example of Fig. 8, the printed circuit board 210 is mounted to the base layer 112 by a mechanical bond 212 (e.g. adhesive) on the opposite side from the nanopore layer 110. Thus, the printed circuit board 210 is disposed outside the outlet chamber 108, as shown in Fig. 8. The sense layer 144 is connected to the printed circuit board 210 by a wire bond 214, or any other suitable electrical connection. The nanopore layer 110 has a smaller area than the sense layer 144 to provide space for the wire bond 214.

In the example of Fig. 9, the printed circuit board 210 is mounted to the sense layer 144 by a solder bump connection 222 (e.g. adhesive) on the same side as the nanopore layer 110. Thus the nanopore layer 110 has a smaller area than the sense layer 144 to provide space for the solder bump connection 222. The solder bump connection 222 provides both mechanical and electrical connection between the printed circuit board 210 and the sense layer 144.

In the example of Fig. 9, the analyte reservoir 106 and the outlet chamber 108 are each formed in respective flowcells 224, 226 which may be made of any suitable material, for example plastic. The flowcells 224, 226 allow flow of fluid into and out of the analyte reservoir 106 and the outlet chamber 108.

The flowcell 224 that forms the analyte reservoir 106 is sealed to the printed circuit board 210 around the analyte reservoir 106 by a gasket 228, and the printed circuit board 210 is sealed to the edges of the nanopore layer 110 around the analyte reservoir 106 by a sealant 230.

The flowcell 224 that forms the outlet chamber 108 is sealed to the base layer 112 around the outlet chamber 108 by a gasket 232.

The examples of Figs. 8 and 9 can be modified in various ways, for example to provide sealing in other locations (e.g. around the outside edge of the base layer 112) and by any suitable means.

The electrical model of a nanopore sensor 102 has been described above. More generally, a voltage source, not shown in Fig. 2, applies a potential difference between the drive electrodes 132 configured in the analyte reservoir 106 and outlet chamber 108. The drive electrodes 132 impose an electrical potential across the passage 114, including the nanopore 116 and channel 122. The nanopore resistance and channel resistance are significantly higher than the overall fluidic resistance of the analyte reservoir 106 and outlet chamber 108 and, therefore, the nanopore 116 and channel 122 are the dominant components in an equivalent electrical circuit. As shown in Fig. 2, the sensor electrode 126 lies between the nanopore 116 and channel 122 such that it can sense the fluidic electrical potential at the electrical transduction element in the passage 114. In other words, the sensor electrode 126 can sense a signal indicative of local electrical potential fluctuations in the passage 114. Although the configuration in Fig. 2 is an example, the sensor electrode 126 can be located in the analyte reservoir 106 or outlet chamber 108. The sensor electrode 126 can function as the base or gate of a transistor device for measuring electrical potential of the fluid at the location of the sensor electrode 126 when a fluid is provided in the passage 114. The sensor electrode 126 can detect fluctuations in voltage as a species object, such as a strand of DNA, translocates through the nanopore 116.

The embodiments herein have described a device 149 having a single analyte reservoir 106 separated from a single outlet chamber 108 by the structure 100. In light of the teaching herein alternative arrangements can be implemented and include a device 149 having (i) two or more analyte reservoirs 106 separated from a common outlet chamber 108 by the structure 100, (ii) a common analyte reservoir 106 separated from two or more outlet chambers 108 by the structure 100, or (iii) two or more analyte reservoirs 106 separated from two or more respective outlet chambers 108 by the structure 100.

The nanopore layer 110 can be formed separately having an array of wells 142 that can be formed in a number of ways, one of which is by lithographically patterning a polymer layer. The wells 142 in the nanopore layer 110 are then aligned with the channels 122 of the base layer 112 such that each nanopore sensor 102 has a passage 114 defined by the well 142 and channel 122. The well 142 shown in Fig. 2 is substantial in comparison to the nanopore 116 located in the membrane 118. The nanopore 116 of Fig. 2 is a biological nanopore in a membrane 118 such as an amphiphilic membrane. Alternatively, each nanopore 116 can be a solid state nanopore located in a solid-state membrane. Further alternatively, the nanopores 116 can be biological nanopores located in a solid-state membrane. In light of the dimensions of the nanopore 116 relative to the width of the channel 122, which is greater in diameter, a well 142 can be said to form beneath the nanopore 116. The nanopore 116, therefore, defines a part of the passage 114 in each of the alternative nanopore configurations.

Any membrane 118 may be used in accordance with various aspects described herein. Suitable membranes 118 are well-known in the art. The membrane 118 can be an amphiphilic layer or a solid-state layer. An amphiphilic layer is a layer formed from amphiphilic molecules, such as phospholipids, which have both hydrophilic and lipophilic properties. The amphiphilic molecules may be synthetic or naturally occurring. Non-naturally occurring amphiphiles and amphiphiles which form a monolayer are known in the art and include, for example, block copolymers (Gonzalez-Perez et al., Langmuir, 2009, 25, 10447-10450). The copolymer may be a triblock, tetrablock or pentablock copolymer. The membrane 118 can be a triblock or diblock copolymer membrane.

Membranes 118 formed from block copolymers hold several advantages over biological lipid membranes. Because the triblock copolymer is synthesized, the exact construction can be carefully controlled to provide the correct chain lengths and properties required to form membranes and to interact with pores and other proteins.

Block copolymers may also be constructed from sub-units that are not classed as lipid sub-materials; for example, a hydrophobic polymer may be made from siloxane or other non-hydrocarbon-based monomers. The hydrophilic sub-section of block copolymer can also possess low protein binding properties, which allows the creation of a membrane 118 that is highly resistant when exposed to raw biological samples. This head group unit may also be derived from non-classical lipid head-groups.

Triblock copolymer membranes also have increased mechanical and environmental stability compared with biological lipid membranes, for example a much higher operational temperature or pH range. The synthetic nature of the block copolymers provides a platform to customize polymer-based membranes for a wide range of applications.

The membrane 118 can be one of the membranes disclosed in WO2014/064443 or WO2014/064444 hereby incorporated by reference in its entirety. These documents also disclose suitable polymers.

The amphiphilic molecules may be chemically-modified or functionalized to facilitate coupling of the polynucleotide.

The amphiphilic layer may be a monolayer or a bilayer. The amphiphilic layer is typically planar. The amphiphilic layer may be curved. The amphiphilic layer may be supported. The amphiphilic layer may be concave. The amphiphilic layer may be suspended from raised pillars such that the peripheral region of the amphiphilic layer (which is attached to the pillars) is higher than the amphiphilic layer region. This may allow the microparticle to travel, move, slide or roll along the membrane as described above.

The membrane 118 may be a lipid bilayer. Suitable lipid bilayers are disclosed in WO 2008/102121, WO 2009/077734 and WO 2006/100484.

Methods for forming lipid bilayers are known in the art. Lipid bilayers are commonly formed by the method of Montal and Mueller (Proc. Natl. Acad. Sci. USA., 1972; 69: 3561-3566), in which a lipid monolayer is carried on aqueous solution/air interface past either side of an aperture which is perpendicular to that interface.

Solid state layers can be formed from both organic and inorganic materials including, but not limited to, microelectronic materials, insulating materials such as Si₃N₄, Al₂O₃, and SiO, organic and inorganic polymers such as polyamide, plastics such as Teflon^{®} or elastomers such as two-component addition-cure silicone rubber, and glasses. The solid-state layer may be formed from graphene. Suitable graphene layers are disclosed in WO 2009/035647. Yusko et al., Nature Nanotechnology, 2011; 6: 253-260 and US Patent Application No. 2013/0048499 describe the delivery of proteins to transmembrane pores in solid state layers without the use of microparticles.

Any transmembrane pore may be used. The nanopore 116 may be biological or artificial. Suitable nanopores 116 include, but are not limited to, protein pores, polynucleotide pores and solid-state pores. The nanopore 116 may be a DNA origami pore (Langecker et al., Science, 2012; 338: 932-936).

The transmembrane pore can be a transmembrane protein pore. A transmembrane protein pore is a polypeptide or a collection of polypeptides that permits hydrated ions, such as the byproducts of processing a polynucleotide with a polymerase, to flow from one side of a membrane 118 to the other side of the membrane 118. In one embodiment, the transmembrane protein pore is capable of forming a nanopore 116 that permits hydrated ions driven by an applied potential to flow from one side of the membrane 118 to the other. The transmembrane protein pore can permit polynucleotides to flow from one side of the membrane 118, such as a triblock copolymer membrane, to the other. The transmembrane protein pore may allow a polynucleotide, such as DNA or RNA, to be moved through the nanopore 116.

The transmembrane protein pore may be a monomer or an oligomer. The pore can be made up of several repeating subunits, such as at least 6, at least 7, at least 8, at least 9, at least 10, at least 11, at least 12, at least 13, at least 14, at least 15, or at least 16 subunits. The pore can be a hexameric, heptameric, octameric or nonameric pore. The pore may be a homo-oligomer or a hetero-oligomer.

The transmembrane protein pore typically comprises a barrel or channel through which the ions may flow. The subunits of the pore typically surround a central axis and contribute strands to a transmembrane β barrel or channel or a transmembrane α-helix bundle or channel. The barrel or channel of the transmembrane protein pore typically comprises amino acids that facilitate interaction with nucleotides, polynucleotides or nucleic acids. These amino acids can be located near a constriction of the barrel or channel. The transmembrane protein pore typically comprises one or more positively charged amino acids, such as arginine, lysine or histidine, or aromatic amino acids, such as tyrosine or tryptophan. These amino acids typically facilitate the interaction between the pore and nucleotides, polynucleotides or nucleic acids.

Transmembrane protein pores for use in accordance with the invention can be derived from β-barrel pores or α-helix bundle pores. The transmembrane pore may be derived from or based on Msp, α-hemolysin (α-HL), lysenin, CsgG, ClyA, Sp1 and hemolytic protein fragaceatoxin C ( FraC). The transmembrane protein pore can be derived from CsgG. Suitable pores derived from CsgG are disclosed in WO 2016/034591. The transmembrane pore may be derived from lysenin. Suitable pores derived from lysenin are disclosed in WO 2013/153359.

The analytes (including, e.g., proteins, peptides, small molecules, polypeptide, polynucleotides) may be present in an analyte. The analyte may be any suitable sample. The analyte may be a biological sample. Any embodiment of the methods described herein may be carried out *in vitro* on an analyte obtained from or extracted from any organism or microorganism. The organism or microorganism is typically archaean, prokaryotic or eukaryotic and typically belongs to one of the five kingdoms: plantae, animalia, fungi, monera and protista. In some embodiments, the methods of various aspects described herein may be carried out *in vitro* on an analyte obtained from or extracted from any virus.

The analyte can be a fluid sample. The analyte can comprise a body fluid. The body fluid may be obtained from a human or animal. The human or animal may have, be suspected of having or be at risk of a disease. The analyte may be urine, lymph, saliva, mucus, seminal fluid or amniotic fluid, but can be whole blood, plasma or serum. Typically, the analyte is human in origin, but alternatively it may be from another mammal such as from commercially farmed animals such as horses, cattle, sheep or pigs or may alternatively be pets such as cats or dogs.
Alternatively, an analyte can be of plant origin.

The analyte may be a non-biological sample. The non-biological sample can be a fluid sample. An ionic salt such as potassium chloride may be added to the sample to effect ion flow through the nanopore.

The polynucleotide may be single stranded or double stranded. At least a portion of the polynucleotide may be double stranded.

The polynucleotide can be a nucleic acid, such as deoxyribonucleic acid (DNA) or ribonucleic acid (RNA). The polynucleotide can comprise one strand of RNA hybridised to one strand of DNA. The polynucleotide may be any synthetic nucleic acid known in the art, such as peptide nucleic acid (PNA), glycerol nucleic acid (GNA), threose nucleic acid (TNA), locked nucleic acid (LNA) or other synthetic polymers with nucleotide side chains. The polynucleotide can be any length.

Any number of polynucleotides can be investigated. For instance, the method may concern characterising 2, 3, 4, 5, 6, 7, 8, 9, 10, 20, 30, 50, 100 or more polynucleotides. If two or more polynucleotides are characterised, they may be different polynucleotides or two instances of the same polynucleotide.

The polynucleotide can be naturally occurring or artificial.

The method may involve measuring two, three, four or five or more characteristics of a polynucleotide. The one or more characteristics can be selected from (i) the length of the polynucleotide, (ii) the identity of the polynucleotide, (iii) the sequence of the polynucleotide, (iv) the secondary structure of the polynucleotide and (v) whether or not the polynucleotide is modified.

For (iii), the sequence of the polynucleotide can be determined as described previously. Suitable sequencing methods, particularly those using electrical measurements, are described in Stoddart D et al., Proc Natl Acad Sci, 12;106(19):7702-7, Lieberman KR et al, J Am Chem Soc. 2010;132(50):17961-72, and International Application WO 2000/28312.

The secondary structure may be measured in a variety of ways. For instance, if the method involves an electrical measurement, the secondary structure may be measured using a change in dwell time or a change in ion current flowing through the pore. This allows regions of single-stranded and double-stranded polynucleotide to be distinguished.

The presence or absence of any modification may be measured. The method can comprises determining whether or not the polynucleotide is modified by methylation, by oxidation, by damage, with one or more proteins or with one or more labels, tags or spacers. Specific modifications will result in specific interactions with the pore which can be measured using the methods described below.

In some embodiments of various aspects described herein, the method may involve further characterizing the target polynucleotide. As the target polynucleotide is contacted with the pore, one or more measurements which are indicative of one or more characteristics of the target polynucleotide are taken as the polynucleotide moves with respect to the pore.

The method may involve determining whether or not the polynucleotide is modified. The presence or absence of any modification may be measured. The method can comprises determining whether or not the polynucleotide is modified by methylation, by oxidation, by damage, with one or more proteins or with one or more labels, tags or spacers.

Also provided is a kit for characterising a target polynucleotide. The kit comprises a pore as disclosed herein and the components of a membrane. The membrane can be formed from the components. The pore can be present in the membrane. The kit may comprise components of any of the membranes disclosed above, such as an amphiphilic layer or a triblock copolymer membrane.

### List of features:

| | |
|---|---|
| 2 | Sensor device |
| 4 | Solid-state nanopore |
| 6 | Analyte |
| 8 | Body |
| 10 | Cis reservoir |
| 12 | Fluidic passage |
| 14 | Trans reservoir |
| 16 | Sensor |
| 18 | Electrodes |
| 100 | Structure |
| 101 | Pixel |
| 102 | Nanopore structure |
| 102a | Sensor module |
| 104 | Array of nanopore structures |
| 106 | Analyte reservoir |
| 108 | Outlet chamber |
| 110 | Nanopore layer |
| 112 | Base layer |
| 114 | Passage |
| 116 | Nanopore |
| 118 | Membrane |
| 120 | First end |
| 122 | Channel |
| 122a | Blocked channel |
| 124 | Second end |
| 126 | sensor electrode |
| 128 | Connection |
| 130 | Electronic circuit |
| 132 | Drive electrodes |
| 132a | Cis electrode |
| 132b | Trans electrode |
| 134 | Cis-surface |
| 136 | Cis-plane |
| 138 | Trans-surface |
| 140 | Trans-plane |
| 142 | Well |
| 142a | Well aperture |
| 144 | Sense layer |
| 146 | Cavity |
| 148 | Sensor aperture |
| 149 | Device |
| 150 | Integrated circuit |
| 151 | Connector |
| 152 | Sensing Circuit |
| 153 | Sensing Transistor |
| 154 | Sensor terminal |
| 155 | Control Circuit |
| 156 | Control Terminal |
| 160 | Compensation circuit |
| 161 | Resistor Model |
| 162 | Capacitor |
| 164 | Flicking switch |
| 166 | Guard switch |
| 168 | Inline amplifier |
| 170 | Feedback amplifier |
| 172 | Compensation capacitor |
| 174 | Row-column readout circuit |
| 176 | Row decoder |
| 178 | Column readout |
| 180 | ADC |
| 182 | Control electrode |
| 184 | Guard conductor |
| 192 | Oxide layer |
| 201 | First Schematic Circuit |
| 202 | Second Schematic Circuit |
| 203 | First Conductive Element |
| 204 | Second Conductive Element |
| 205 | Buffer |
| 210 | Printed Circuit Board |
| 212 | Mechanical Bond |
| 214 | Wire Bond |
| 216,218 | Gaskets |
| 222 | Solder Bump Connection |
| 224,226 | Flowcells |
| 228 | Gasket |
| 230 | Sealant |
| 232 | Gasket |

## Claims

1. A device having an array of nanopore structures configured in a sheet, the sheet comprising:
a nanopore layer (110) having an array of nanopores (116) and an array of wells (142), each well of the array of wells supporting a membrane (118) that spans the well and having a respective nanopore (116) of the array of nanopores (116) provided therein; and
a base layer (112) having an array of channels (122), said base layer (112) sandwiched or laminated to the nanopore layer (110) to form the sheet, wherein the nanopores (116) and the wells (142) are aligned with the channels (122),
wherein each of the nanopore structures comprise a passage (114), each passage defined at least in part by:
one of the nanopores (116) and one of the wells (142) of the nanopore layer (110), at one side of the passage;
a channel (122) of the base layer (112) at the other side of the passage; and
an electrical transduction element (126), and
**characterised in that** each nanopore structure of the array has a well (142) formed at a first end of the passage, and a nanopore (116) is configured at the first end of the well (142), and wherein the electrical transduction element (126) is configured on the opposite side of the well (142) to the nanopore (116).

2. The device of claim 1, wherein the electrical transduction element (126) in each passage is disposed between the nanopore layer (110) and at least a portion of the channel (122), and optionally configured with a connection for measuring electrical potential of a fluid at the location of the electrical transduction element (126) when the fluid is provided in the passage.

3. The device of claim 1 or 2, further comprising: an analyte reservoir (106) and an outlet chamber (108) separated, at least in part, by the sheet, wherein the sheet has an array of the passages disposed between the analyte reservoir (106) and the outlet chamber (108) to connect the analyte reservoir (106) to the outlet chamber (108).

4. The device of any preceding claim, wherein the sheet is substantially planar and has a cis-surface on the nanopore layer (110) for facing an analyte reservoir (106) and defining a cis-plane, and a trans-surface of the base layer (112) for facing an outlet chamber (108) and defining a trans-plane, and the electrical transduction elements (126) of the array are embedded, at least in part, within the sheet between the cis-plane and the trans-plane.

5. The device of any preceding claim, wherein the electrical transduction elements (126) of the array are sandwiched between the nanopore layer (110) and the base layer (112).

6. The device of any preceding claim, wherein:
the membrane (118) comprises a solid-state membrane and the nanopore (116) is a solid-state nanopore or a biological nanopore; or
the membrane (118) comprises an amphiphilic membrane and the nanopore (116) is a biological nanopore.

7. The device of any preceding claim, further comprising a sense layer (144) having an array of the electrical transduction elements (126), wherein said sense layer (144) is sandwiched between the nanopore layer (110) and the base layer (112),
wherein, optionally, the electrical transduction element (126) is formed upon the sense layer (144), the electrical transduction element (126) having (i) an exposed portion for connection to a fluid in the passage, and (ii) an embedded portion embedded within the sheet, and/or (iii) a connection portion for connection to a measurement circuit separate from the sheet.

8. The device of any of any preceding claim, wherein the electrical transduction element (126) covers (i) at least in part, a wall of the passage, and/or (ii) in cross-section, a portion of a wall of the channel (122).

9. The device of any preceding claim, wherein the electrical transduction element (126) forms an annulus around the passage.

10. The device of any preceding claim, further comprising an analyte reservoir (106), wherein the electrical transduction element (126) forms, at least in part, the surface of the base layer (112) or the sense layer (144) around the passage and has an exposed portion arranged to face the analyte reservoir (106).

11. The device of any preceding claim, further comprising an outlet chamber (108), wherein the electrical transduction element (126) forms, at least in part, the surface of the sense layer (144) around the passage and has an exposed portion arranged to face the outlet chamber (108),
wherein, optionally, the exposed portion forms part of a wall of a cavity formed in the sense layer (144) between the well (142) and the channel (122).

12. The device of any preceding claim, wherein the array of nanopore structures are arranged in (i) a two-dimensional matrix, and/or (ii) a tessellated pattern.

13. The device of any preceding claim, wherein the array of nanopore structures are configured for nanopore sensing.

14. A method of forming a device having an array of nanopore structures configured in a sheet, including arranging the sheet to separate an analyte reservoir (106) and an outlet chamber (108) of the device such that each nanopore structure provides a passage for fluid connection through the structure between the analyte reservoir (106) and outlet chamber (108), the method comprising:
forming a nanopore layer (110) having an array of nanopores (116) and an array of wells (142) by providing a membrane (118) across each well (142) of the array of wells (142) and inserting a respective nanopore (116) of the array of nanopores (116) in said membrane (118);
forming an array of electrical transduction elements (126);
forming a base layer (112) having an array of channels (122), said base layer (112) sandwiched or laminated to the nanopore layer (110) to form the sheet such that the nanopores (116) and the wells (142) are aligned with the electrical transduction elements (126) and channels (122); and
providing a passage (114) through each of the nanopore structures such that each passage is defined at least in part by:
one of the nanopores (116) and one of the wells (142) of the nanopore layer (110), at one side of the passage;
a channel (122) of the base layer (112) at the other side of the passage; and
an electrical transduction element (126), and
**characterised in that** each nanopore structure of the array has a well (142) formed at a first end of the passage, and a nanopore (116) is configured at the first end of the well (142), and wherein the electrical transduction element (126) is configured on the opposite side of the well (142) to the nanopore (116).

15. The method according to claim 14, wherein aligning the nanopore layer (110), base layer (112) and array of electrical transduction elements (126) includes sandwiching the array of electrical transduction elements (126) between the nanopore layer (110) and the base layer (112).

16. The method according to claim 14 or 15, further comprising forming cavities adjacent at least a portion of each of the electrical transduction elements (126).

17. The method according to any of claims 14 to 16, further comprising:
forming the array of electrical transduction elements (126) on a sense layer (144); and
sandwiching the sense layer (144) between the nanopore layer (110) and the base layer (112).

18. The method according to claim to any of claims 14 to 17, further comprising arranging the electrical transduction element (126) to have:
(i) an exposed portion for connection to a fluid in the passage, and
(ii) an embedded portion embedded within the structure, and/or
(iii) a connection portion for connection to a measurement circuit separate from the structure.

19. The method according to any of claims 14 to 18, wherein the nanopore layer (110) is removably attachable to the structure and the method further comprises removing the nanopore layer (110) and replacing it with another nanopore layer.

## Patentansprüche

1. Vorrichtung mit einer Anordnung von Nanoporenstrukturen, die in einer Folie konfiguriert sind, wobei die Folie umfasst:
eine Nanoporenschicht (110), die eine Anordnung von Nanoporen (116) und eine Anordnung von Vertiefungen (142) aufweist, wobei jede Vertiefung der Anordnung von Vertiefungen eine Membran (118) trägt, welche die Vertiefung überspannt und eine jeweilige Nanopore (116) der darin bereitgestellten Anordnung von Nanoporen (116) aufweist: und
eine Basisschicht (112), die eine Anordnung von Kanälen (122) aufweist, wobei die Basisschicht (112) eingeschoben oder auf die Nanoporenschicht (110) laminiert ist, um die Folie zu bilden, wobei die Nanoporen (116) und die Vertiefungen (142) auf die Kanäle (122) ausgerichtet sind,
wobei jede der Nanoporenstrukturen einen Durchgang (114) umfasst, wobei jeder Durchgang mindestens teilweise definiert ist durch:
eine der Nanoporen (116) und eine der Vertiefungen (142) der Nanoporenschicht (110) auf einer Seite des Durchgangs;
einen Kanal (122) der Basisschicht (112) auf der anderen Seite des Durchgangs; und
ein elektrisches Transduktionselement (126), und
**dadurch gekennzeichnet, dass** jede Nanoporenstruktur der Anordnung eine Vertiefung (142) aufweist, die an einem ersten Ende des Durchgangs gebildet ist, und eine Nanopore (116) an dem ersten Ende der Vertiefung (142) konfiguriert ist, und wobei das elektrische Transduktionselement (126) auf der Seite der Vertiefung (142) gegenüber der Nanopore (116) konfiguriert ist.

2. Vorrichtung nach Anspruch 1, wobei das elektrische Transduktionselement (126) in jedem Durchgang zwischen der Nanoporenschicht (110) und mindestens einem Abschnitt des Kanals (122) angeordnet ist, und wahlweise mit einer Verbindung konfiguriert ist, um ein elektrisches Potential eines Fluids an der Stelle des elektrischen Transduktionselements (126) zu messen, wenn das Fluid in dem Durchgang bereitgestellt wird.

3. Vorrichtung nach Anspruch 1 oder 2, ferner umfassend: ein Analytenreservoir (106) und eine Auslasskammer (108), die mindestens teilweise durch die Folie getrennt ist, wobei die Folie eine Anordnung der Durchgänge aufweist, die zwischen dem Analytenreservoir (106) und der Auslasskammer (108) angeordnet sind, um das Analytenreservoir (106) mit der Auslasskammer (108) zu verbinden.

4. Vorrichtung nach einem der vorhergehenden Ansprüche, wobei die Folie im Wesentlichen eben ist und eine Cis-Oberfläche auf der Nanoporenschicht (110), um einem Analytenreservoir (106) gegenüberzuliegen und eine Cis-Ebene zu definieren, und eine Trans-Oberfläche der Basisschicht (112), um einer Auslasskammer (108) gegenüberzuliegen und eine Trans-Ebene zu definieren, aufweist, und die elektrischen Transduktionselemente (126) der Anordnung mindestens teilweise innerhalb der Folie zwischen der Cis-Ebene und der Trans-Ebene eingebettet sind.

5. Vorrichtung nach einem der vorhergehenden Ansprüche, wobei die elektrischen Transduktionselemente (126) der Anordnung zwischen der Nanoporenschicht (110) und der Basisschicht (112) eingeschoben sind.

6. Vorrichtung nach einem der vorhergehenden Ansprüche, wobei:
die Membran (118) eine Festkörper-Membran umfasst, und die Nanopore (116) eine Festkörper-Nanopore oder eine biologische Nanopore ist; oder
die Membran (118) eine amphiphile Membran ist, und die Nanopore (116) eine biologische Nanopore ist.

7. Vorrichtung nach einem der vorhergehenden Ansprüche, ferner umfassend eine Detektionsschicht (144), die eine Anordnung der elektrischen Transduktionselemente (126) aufweist, wobei die Detektionsschicht (144) zwischen der Nanoporenschicht (110) und der Basisschicht (112) eingeschoben ist,
wobei wahlweise das elektrische Transduktionselement (126) auf der Detektionsschicht (144) gebildet ist, wobei das elektrische Transduktionselement (126) (i) einen freigelegten Abschnitt zur Verbindung mit einem Fluid in dem Durchgang und (ii) einen eingebetteten Abschnitt, der innerhalb der Folie eingebettet ist, und/oder (iii) einen Verbindungsabschnitt zur Verbindung mit einer Messschaltung, die von der Folie getrennt ist, aufweist.

8. Vorrichtung nach einem der vorhergehenden Ansprüche, wobei das elektrische Transduktionselement (126) (i) mindestens teilweise eine Wand des Durchgangs und/oder (ii) im Querschnitt einen Abschnitt einer Wand des Kanals (122) abdeckt.

9. Vorrichtung nach einem der vorhergehenden Ansprüche, wobei das elektrische Transduktionselement (126) einen Ringraum um den Durchgang herum bildet.

10. Vorrichtung nach einem der vorhergehenden Ansprüche, ferner umfassend ein Analytenreservoir (106), wobei das elektrische Transduktionselement (126) mindestens teilweise die Oberfläche der Basisschicht (112) oder der Detektionsschicht (144) um den Durchgang herum bildet und einen freigelegten Abschnitt aufweist, der dazu angeordnet ist, dem Analytenreservoir (106) gegenüberzuliegen.

11. Vorrichtung nach einem der vorhergehenden Ansprüche, ferner umfassend eine Auslasskammer (108), wobei das elektrische Transduktionselement (126) mindestens teilweise die Oberfläche der Detektionsschicht (144) um den Durchgang herum bildet und einen freigelegten Abschnitt aufweist, der dazu angeordnet ist, der Auslasskammer (108) gegenüberzuliegen,
wobei wahlweise der freigelegte Abschnitt Teil einer Wand eines Hohlraums ist, der in der Detektionsschicht (144) zwischen der Vertiefung (142) und dem Kanal (122) gebildet ist.

12. Vorrichtung nach einem der vorhergehenden Ansprüche, wobei die Anordnung von Nanoporenstrukturen in (i) einer zweidimensionalen Matrix und/oder (ii) einem tessellierten Motiv angeordnet ist.

13. Vorrichtung nach einem der vorhergehenden Ansprüche, wobei die Anordnung von Nanoporenstrukturen zur Detektion von Nanoporen konfiguriert ist.

14. Verfahren zum Bilden einer Vorrichtung, die eine Anordnung von Nanoporenstrukturen aufweist, die in einer Folie konfiguriert sind, umfassend das Anordnen der Folie zum Trennen eines Analytenreservoirs (106) und einer Auslasskammer (108) der Vorrichtung, so dass jede Nanoporenstruktur einen Durchgang zur Fluidverbindung durch die Struktur hindurch zwischen dem Analytenreservoir (106) und der Auslasskammer (108) bereitstellt, wobei das Verfahren umfasst:
Bilden einer Nanoporenschicht (110), die eine Anordnung von Nanoporen (116) und eine Anordnung von Vertiefungen (142) aufweist, indem eine Membran (118) über jede Vertiefung (142) der Anordnung von Vertiefungen (142) bereitgestellt wird und eine jeweilige Nanopore (116) der Anordnung von Nanoporen (116) in die Membran (118) eingefügt wird;
Bilden einer Anordnung von elektrischen Transduktionselementen (126);
Bilden einer Basisschicht (112), die eine Anordnung von Kanälen (122) aufweist, wobei die Basisschicht (112) eingeschoben oder auf die Nanoporenschicht (110) laminiert wird, um die Folie zu bilden, so dass die Nanoporen (116) und die Vertiefungen (142) auf die elektrischen Transduktionselemente (126) und die Kanäle (122) ausgerichtet sind; und
Bereitstellen eines Durchgangs (114) durch jede der Nanoporenstrukturen hindurch, so dass jeder Durchgang mindestens teilweise definiert ist durch:
eine der Nanoporen (116) und eine der Vertiefungen (142) der Nanoporenschicht (110) auf einer Seite des Durchgangs;
einen Kanal (122) der Basisschicht (112) auf der anderen Seite des Durchgangs; und
ein elektrisches Transduktionselement (126), und
**dadurch gekennzeichnet, dass** jede Nanoporenstruktur der Anordnung eine Vertiefung (142) aufweist, die an einem ersten Ende des Durchgangs gebildet ist, und eine Nanopore (116) an dem ersten Ende der Vertiefung (142) konfiguriert ist, und wobei das elektrische Transduktionselement (126) auf der Seite der Vertiefungen (142) gegenüber der Nanopore (116) konfiguriert ist.

15. Verfahren nach Anspruch 14, wobei das Ausrichten der Nanoporenschicht (110), der Basisschicht (112) und der Anordnung von elektrischen Transduktionselementen (126) das Einschieben der Anordnung von elektrischen Transduktionselementen (126) zwischen die Nanoporenschicht (110) und die Basisschicht (112) umfasst.

16. Verfahren nach Anspruch 14 oder 15, ferner umfassend das Bilden von Hohlräumen, die an mindestens einen Abschnitt jedes der elektrischen Transduktionselemente (126) angrenzend sind.

17. Verfahren nach einem der vorhergehenden Ansprüche 14 bis 16, ferner umfassend:
Bilden der Anordnung von elektrischen Transduktionselementen (126) auf einer Detektionsschicht (144); und
Einschieben der Detektionsschicht (144) zwischen die Nanoporenschicht (110) und die Basisschicht (112).

18. Verfahren nach einem der vorhergehenden Ansprüche 14 bis 17, ferner umfassend das Anordnen des elektrischen Transduktionselements (126), so dass es aufweist:
(i) einen freigelegten Abschnitt zur Verbindung mit einem Fluid in dem Durchgang, und
(ii) einen eingebetteten Abschnitt, der innerhalb der Struktur eingebettet ist, und/oder
(iii) einen Verbindungsabschnitt zur Verbindung mit einer Messschaltung, die von der Struktur getrennt ist.

19. Verfahren nach einem der vorhergehenden Ansprüche 14 bis 18, wobei die Nanoporenschicht (110) an der Struktur abnehmbar angebracht werden kann, und das Verfahren ferner das Entfernen der Nanoporenschicht (110) und das Ersetzen derselben durch eine andere Nanoporenschicht umfasst.

## Revendications

1. Dispositif présentant un réseau de structures à nanopores configurées dans une feuille, la feuille comprenant :
une couche de nanopores (110) présentant un réseau de nanopores (116) et un réseau de puits (142), chaque puits du réseau de puits supportant une membrane (118) qui couvre le puits et présente un nanopore (116) respectif du réseau de nanopores (116) fourni dans celle-ci : et
une couche de base (112) présentant un réseau de canaux (122), ladite couche de base (112) étant interposée ou laminée sur la couche de nanopores (110) pour former la feuille, les nanopores (116) et les puits (142) étant alignés sur les canaux (122),
dans lequel chacune des structures à nanopores comprend un passage (114), chaque passage étant défini au moins en partie par :
l'un des nanopores (116) et l'un des puits (142) de la couche de nanopores (110), d'un côté du passage ;
un canal (122) de la couche de base (112) de l'autre côté du passage ; et
un élément de transduction électrique (126), et
**caractérisé en ce que** chaque structure à nanopores du réseau présente un puits (142) formé à une première extrémité du passage, et un nanopore (116) est configuré à la première extrémité du puits (142), et dans lequel l'élément de transduction électrique (126) est configuré du côté du puits (142) opposé au nanopore (116).

2. Dispositif selon la revendication 1, dans lequel l'élément de transduction électrique (126) dans chaque passage est disposé entre la couche de nanopores (110) et au moins une partie du canal (122), et en option configuré avec une connexion pour mesurer un potentiel électrique d'un fluide à l'emplacement de l'élément de transduction électrique (126) lorsque le fluide est fourni dans le passage.

3. Dispositif selon la revendication 1 ou 2, comprenant en outre : un réservoir d'analyte (106) et une chambre de sortie (108) séparée, au moins en partie, par la feuille, la feuille présentant un réseau des passages disposé entre le réservoir d'analyte (106) et la chambre de sortie (108) pour connecter le réservoir d'analyte (106) à la chambre de sortie (108).

4. Dispositif selon l'une quelconque des revendications précédentes, dans lequel la feuille est substantiellement planaire et présente une surface cis sur la couche de nanopores (110) pour faire face à un réservoir d'analyte (106) et définir un plan cis, et une surface trans de la couche de base (112) pour faire face à une chambre de sortie (108) et définir un plan trans, et les éléments de transduction électrique (126) du réseau sont incorporés, au moins en partie, à l'intérieur de la feuille entre le plan cis et le plan trans.

5. Dispositif selon l'une quelconque des revendications précédentes, dans lequel les éléments de transduction électrique (126) du réseau sont interposés entre la couche de nanopores (110) et la couche de base (112).

6. Dispositif selon l'une quelconque des revendications précédentes, dans lequel :
la membrane (118) comprend une membrane à l'état solide et le nanopore (116) est un nanopore à l'état solide ou un nanopore biologique ; ou
la membrane (118) comprend une membrane amphiphile et le nanopore (116) est un nanopore biologique.

7. Dispositif selon l'une quelconque des revendications précédentes, comprenant en outre une couche de détection (144) présentant un réseau des éléments de transduction électrique (126), dans lequel ladite couche de détection (144) est intercalée entre la couche de nanopores (110) et la couche de base (112),
dans lequel, en option, l'élément de transduction électrique (126) est formé sur la couche de détection (144), l'élément de transduction électrique (126) présentant (i) une partie exposée pour la connexion à un fluide dans le passage, et (ii) une partie incorporée, incorporée à l'intérieur de la feuille, et/ou (iii) une partie de connexion pour la connexion à un circuit de mesure séparé de la feuille.

8. Dispositif selon l'une quelconque des revendications précédentes, dans lequel l'élément de transduction électrique (126) couvre (i) au moins en partie une paroi du passage, et/ou (ii) en coupe transversale une partie d'une paroi du canal (122).

9. Dispositif selon l'une quelconque des revendications précédentes, dans lequel l'élément de transduction électrique (126) forme un espace annulaire autour du passage.

10. Dispositif selon l'une quelconque des revendications précédentes, comprenant en outre un réservoir d'analyte (106), dans lequel l'élément de transduction électrique (126) forme, au moins en partie, la surface de la couche de base (112) ou de la couche de détection (144) autour du passage et présente une partie exposée agencée pour faire face au réservoir d'analyte (106).

11. Dispositif selon l'une quelconque des revendications précédentes, comprenant en outre une chambre de sortie (108), dans lequel l'élément de transduction électrique (126) forme, au moins en partie, la surface de la couche de détection (144) autour du passage et présente une partie exposée agencée pour faire face à la chambre de sortie (108),
dans lequel, en option, la partie exposée fait partie d'une paroi d'une cavité formée dans la couche de détection (144) entre le puits (142) et le canal (122).

12. Dispositif selon l'une quelconque des revendications précédentes, dans lequel le réseau de structures à nanopores est agencé dans (i) une matrice bidimensionnelle, et/ou (ii) un motif tessellé.

13. Dispositif selon l'une quelconque des revendications précédentes, dans lequel le réseau de structures à nanopores est configuré pour la détection de nanopores.

14. Procédé permettant de former un dispositif présentant un réseau de structures à nanopores configurées dans une feuille, comprenant l'agencement de la feuille pour séparer un réservoir d'analyte (106) et une chambre de sortie (108) du dispositif de sorte que chaque structure à nanopores fournit un passage pour une connexion de fluide à travers la structure entre le réservoir d'analyte (106) et la chambre de sortie (108), le procédé comprenant les étapes consistant à :
former une couche de nanopores (110) présentant un réseau de nanopores (116) et un réseau de puits (142) en fournissant une membrane (118) sur chaque puits (142) du réseau de puits (142) et en insérant un nanopore (116) respectif du réseau de nanopores (116) dans ladite membrane (118) ;
former un réseau d'éléments de transduction électrique (126) ;
former une couche de base (112) présentant un réseau de canaux (122), ladite couche de base (112) étant intercalée ou laminée sur la couche de nanopores (110) pour former la feuille de sorte que les nanopores (116) et les puits (142) sont alignés sur les éléments de transduction électrique (126) et les canaux (122) ; et
fournir un passage (114) à travers chacune des structures à nanopores de sorte que chaque passage est défini au moins en partie par :
l'un des nanopores (116) et l'un des puits (142) de la couche de nanopores (110), d'un côté du passage ;
un canal (122) de la couche de base (112) de l'autre côté du passage ; et
un élément de transduction électrique (126), et
**caractérisé en ce que** chaque structure à nanopores du réseau présente un puits (142) formé à une première extrémité du passage, et un nanopore (116) est configuré à la première extrémité du puits (142), et dans lequel l'élément de transduction électrique (126) est configuré du côté du puits (142) opposé au nanopore (116).

15. Procédé selon la revendication 14, dans lequel l'alignement de la couche de nanopores (110), de la couche de base (112) et du réseau d'éléments de transduction électrique (126) comprend le fait d'intercaler le réseau d'éléments de transduction électrique (126) entre la couche de nanopores (110) et la couche de base (112).

16. Procédé selon la revendication 14 ou 15, comprenant en outre la formation de cavités adjacentes à au moins une partie de chacun des éléments de transduction électrique (126).

17. Procédé selon l'une quelconque des revendications 14 à 16, comprenant en outre les étapes consistant à :
former le réseau d'éléments de transduction électrique (126) sur une couche de détection (144) ; et
intercaler la couche de détection (144) entre la couche de nanopores (110) et la couche de base (112).

18. Procédé selon l'une quelconque des revendications 14 à 17, comprenant en outre l'agencement de l'élément de transduction électrique (126) pour qu'il présente :
(i) une partie exposée pour la connexion à un fluide dans le passage, et
(ii) une partie incorporée, incorporée à l'intérieur de la structure, et/ou
(iii) une partie de connexion pour la connexion à un circuit de mesure séparé de la structure.

19. Procédé selon l'une quelconque des revendications 14 à 18, dans lequel la couche de nanopores (110) peut être attachée de manière amovible à la structure, et le procédé comprend en outre le retrait de la couche de nanopores (110) et son remplacement par une autre couche de nanopores.
